# EUROPEAN PATENT APPLICATION

(11) **EP 4 201 420 A1**
(43) Date of publication of application: **28.06.2023**
(21) Application number: 21216376.0
(22) Date of filing: 21.12.2021
(51) Int. Cl.: A61K 38/17, A61P 31/04

(54) **NON-HUMAN BACTERICIDAL/PERMEABILITY-INCREASING PROTEIN (BPI) FOR THERAPY OF INFECTIONS**

(71) Applicant: Universität Regensburg, 93053 Regensburg (DE)
(72) Inventor: The designation of the inventor has not yet been filed
(74) Representative: Engelhard, Markus

(57) **Abstract**

The present invention relates to bactericidal/permeability-increasing protein (BPI) for use in a method of preventing or treating a sepsis associated with an infection with Gram-negative bacteria, wherein said BPI is non-human BPI or a fragment thereof.

## Description

### FIELD OF THE INVENTION

The present invention relates to bactericidal/permeability-increasing protein (BPI) for use in a method of preventing or treating a sepsis associated with an infection with Gram-negative bacteria, wherein said BPI is non-human BPI or a fragment thereof.

### BACKGROUND OF THE INVENTION

Multi-drug-resistant bacteria are an emerging health burden and a major cause of death. Particularly, blood stream infections with multi-drug resistant (MDR) Gram-negative pathogens are an emerging threat, often leading to sepsis, a systemic inflammatory cascade characterized by high mortality rates. Beside tissue destruction caused by bacterial invasion, the prognosis of patients with Gram-negative infection is determined by a release of bacterial products such bacterial lipopolysaccharide (LPS), a cell wall component of Gram-negative bacteria, also called bacterial endotoxin.

Initiation of sepsis in Gram-negative infections is primarily attributed to the high immunostimulatory potential of LPS. Recognition of LPS by innate immune cells via the toll-like receptor 4 (TLR4)-MD2 complex induces the secretion of a broad pro-inflammatory cytokine response potentially leading to a dysregulated hyperinflammation and the onset of sepsis. Antibiotic treatment decreases the bacterial load due to bactericidal or bacteriostatic effects. However, LPS neutralization is usually not an effect of antibiotic treatment. There remains the need for LPS neutralization to reduce the systemic immune response during sepsis and improve the survival of patients.

Binding of LPS by the neutrophil-derived bactericidal/permeability-increasing protein (BPI) has been proposed to reduce the immunostimulatory potential of LPS on immune cells and additionally exhibit bactericidal effects towards Gram-negative bacteria [1]. A phase III study investigated the effects of adjunctive administration of a recombinant amino-terminal fragment of BPI (rBPI21) in patients suffering from meningococcal disease [2]. Recipients of rBPI21 showed a tendency towards an improved outcome, including a significant lower risk of amputations. There remains the need for highly efficient means for preventing or treating infections with Gram-negative bacteria. Particularly, there is a need to provide compounds for efficiently preventing or treating a sepsis associated with Gram-negative infections.

### SUMMARY OF THE INVENTION

In the following, the elements of the invention will be described. These elements are listed with specific embodiments. However, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to bactericidal/permeability increasing protein (BPI) for use in a method of preventing or treating a sepsis associated with an infection with Gram-negative bacteria, wherein said BPI is non-human BPI or a fragment thereof.

In one embodiment, said BPI is non-human vertebrate BPI or a fragment thereof; preferably is teleost BPI or a fragment thereof.

In one embodiment, said BPI is BPI of a *Sebastinae* sp. or a fragment thereof; preferably BPI of a *Sebastes* sp. or a fragment thereof; more preferably is BPI of *Sebastes schlegelii* or a fragment thereof.

In one embodiment, said BPI comprises or consists of an amino acid sequence of SEQ ID No. 1.

In one embodiment, said BPI or fragment thereof comprises an N-terminal fragment of BPI, preferably an N-terminal fragment of BPI having an amino acid sequence of SEQ ID No. 2.

In one embodiment, said BPI or fragment thereof comprises an amino acid sequence of any of SEQ ID No. 3-18.

In one embodiment, said BPI comprises a modification selected from
- an amino acid substitution at position 347 of SEQ ID No. 1, preferably an asparagine at position 347 being substituted with alanine;
- one or more amino acid substitutions providing a sequence of any of SEQ ID No. 19-23;
- a modified glycosylation pattern, preferably a removed glycosylation, e.g., at position 347 of SEQ ID No. 1; wherein, optionally, said glycosylation pattern is modified by a mild acid treatment, an amino acid substitution, and/or an enzymatic treatment, preferably a glucosidase treatment e.g., using mannosidase;
and a combination thereof.

In one embodiment, said sepsis is associated with
- a presence of Gram-negative bacteria or component(s) thereof, preferably lipopolysaccharide, in the bloodstream of a patient;
- a dysfunction, preferably failure, of one or more organs of said patient; and/or
- a septic shock.

In one embodiment, in said method, said BPI is administered to a patient in need thereof; preferably administered systemically or locally; more preferably administered intravenously, intravascularly, orally, nasally, mucosally, intrabronchially, intrapulmonarily, intradermally, subcutaneously, intramuscularly, intravascularly, intraperitoneally, intrathecally, intracerebral, intracranial, intraocularly, intraarticularly, intranodally, intratumorally, and/or intrametastatically.

In one embodiment, said patient is characterized by BPI deficiency, e.g., a decreased BPI level and/or a functional BPI deficit; neutropenia; defective neutrophil function; and/or by increased levels of anti-neutrophil cytoplasmic antibodies, preferably anti-BPI autoantibodies.

In one embodiment, said patient is a newborn, a toddler, or a patient having an age of at least 40 years, preferably of at least 60 years.

In one embodiment, said patient suffers from or is at risk of acquiring, in addition to said infection with Gram-negative bacteria, a disease selected from cancer; neutropenia; infections other than said infection with Gram-negative bacteria, e.g., HIV infections; autoimmune diseases, e.g., systemic lupus erythematodes, psoriasis, rheumatic diseases such as rheumatoid arthritis, granulomatosis with polyangiitis, and inflammatory bowel diseases such as Crohn's disease and ulcerative colitis; hematologic diseases; cystic fibrosis; COPD; alpha-1 antitrypsin deficiency; bronchiectasis; TAP deficiency; primary biliary cirrhosis; vasculitis; and immune suppression, e.g., iatrogenic immune suppression and therapeutic immune suppression.

In one embodiment, in said method, said BPI is co-administered with any of
- an antimicrobial agent, preferably selected from antibacterial agents, antifungal agents, virostatic agents, and antiparasitic agents, more preferably an antibiotic; an anti-inflammatory agent, preferably selected from steroids, non-steroidal antiphlogisitic agents, and antibodies targeting a pro-inflammatory cytokine or an immune receptor;
- an immunosuppressive agent, preferably selected from cylosporin, tacrolimus, mycophenolat, and methotrexate;
- an immunotherapeutic agent, preferably selected from anti-TNF antibodies, anti-BAFF antibodies, anti-CD20 antibodies, immune checkpoint inhibitors, TLR ligands, and C-type lectin ligands, e.g., glucans;
and combinations thereof.

In one embodiment, said BPI neutralizes lipopolysaccharide.

In one embodiment, said BPI alleviates said infection and/or said sepsis by neutralizing a lipopolysaccharide of said Gram-negative bacteria.

In a further aspect, the present invention also relates to a method of preventing or treating a sepsis associated with an infection with Gram-negative bacteria, comprising administering to a patient in need thereof an effective amount of BPI, wherein said BPI is non-human BPI or a fragment thereof.

In this aspect, said method of preventing or treating, said sepsis associated with an infection with Gram-negative bacteria, said administering, said patient, and said BPI are as defined herein.

In a further aspect, the present invention also relates to the use of BPI in the manufacture of a medicament for preventing or treating a sepsis associated with an infection with Gram-negative bacteria, wherein said BPI is non-human BPI or a fragment thereof.

In this aspect, said BPI, said preventing or treating, and said sepsis associated with an infection with Gram-negative bacteria, are as defined herein.

### DETAILED DESCRIPTION

The present invention aims at providing a compound to prevent or treat a sepsis associated with Gram-negative bacteria. The inventors have surprisingly found that non-human BPI, particularly non-human vertebrate BPI such as teleost BPI, is highly effective in neutralizing LPS and thereby reducing an inflammatory response towards Gram-negative bacteria. Advantageously, BPI for use of the invention, preferably teleost BPI, is highly effective in neutralizing LPS, thus alleviating a sepsis associated with Gram-negative bacteria.

The term "bactericidal/permeability-increasing protein" or "BPI", as used herein, relates to and includes recombinant, (semi-)synthetic, and natural proteins, biologically active polypeptides and polypeptide fragments, such as a N-terminal fragment of BPI modified in a single amino acid or a N-terminal fragment of BPI, and/or truncated forms having an LPS-neutralizing function, as well as variants and analogs derived thereof. The term also relates to nucleic acids encoding said protein, said polypeptides, said polypeptide fragments, said truncated forms having an LPS-neutralizing function, as well as variants, truncated forms, and analogs derived thereof. As used herein, in some embodiments, the terms "protein" and "peptide" are used interchangeably. Exemplary amino acid sequences of teleost BPI and human BPI are represented by SEQ ID No. 1 and 24, respectively, including polypeptides and polypeptide fragments and truncated forms and other variants thereof retaining at least one of its biological functions, preferably its LPS-neutralizing function. In humans, BPI is a highly expressed protein in neutrophil granulocytes and belongs to the tubular-lipid binding protein (TULIP) family. One of its main functions is the neutralization of the endotoxic activity of bacterial lipopolysaccharides (LPS). Due to its highly affine interaction with lipopolysaccharide, it is bactericidal towards Gram-negative bacteria. Furthermore, BPI triggers opsonization of bacteria by immune cells. Since it exhibits a bactericidal activity against Gram-negative bacteria, blocks LPS-mediated inflammation, and opsonizes bacteria, BPI plays an important role in the innate immune response and therewith in the first line defense in fighting infections. The term "BPI", as used herein, can also refer to modified BPI, wherein BPI has been stabilized by modification, such as of protease cleavage sites, or wherein BPI has other modifications, such as altered glycosylation, e.g., by removed glycosylation sites, as long as such modified form retains an LPS-neutralizing function. Alternatively, BPI can relate to genetic sequences encoding biologically active BPI, and biologically active polypeptide and polypeptide fragments having an LPS-neutralizing function, such as a N-terminal fragment of BPI modified in single amino acids or a N-terminal fragment of BPI, and/or truncated forms, variants, and analogs thereof having an LPS-neutralizing function, which can be applied using a vector or a different delivery system. In a preferred embodiment, BPI is teleost BPI. BPI for use according to the present invention is highly effective in neutralizing LPS. Surprisingly, teleost BPI has higher LPS-neutralizing efficiency than human BPI.

The term "sepsis associated with an infection with Gram-negative bacteria", as used herein, relates to a sepsis correlated with a presence of Gram-negative bacteria or component(s) thereof in a patient's body and/or caused by an infection with Gram-negative bacteria, for example a sepsis occurring when a patient, e.g., a previously healthy individual or an immunocompromised patient, is infected with Gram-negative bacteria. In one embodiment, a sepsis associated with an infection with Gram-negative bacteria comprises any of sepsis, severe sepsis, and/or septic shock. In one embodiment, a sepsis associated with an infection with Gram-negative bacteria comprises an infection, particularly systemic infection, of said patient with Gram-negative bacteria. In one embodiment, said sepsis comprises or consists of severe sepsis and/or a septic shock. In one embodiment, a severe sepsis comprises sepsis-induced organ dysfunction or tissue hypoperfusion. In one embodiment, a septic shock comprises a severe sepsis and persistently low blood pressure. In one embodiment, BPI for use in a method of preventing or treating a sepsis is for use in preventing or treating sepsis, severe sepsis and/or a septic shock. In one embodiment, the sepsis is a life-threatening condition that arises when the body's response to an infection with Gram-negative bacteria causes injury to its own tissues and organs. In one embodiment, said sepsis is associated with
- a presence of Gram-negative bacteria or component(s) thereof, preferably lipopolysaccharide, in a bloodstream of a patient; and/or
- a dysfunction, preferably failure, of one or more organs of said patient; and/or
- a septic shock.

In one embodiment, in said method, prior to an administration of said BPI, a presence of Gram-negative bacteria or component(s) thereof is detected in a blood or tissue sample of said patient. In one embodiment, in said method, said BPI for use is administered, for example preventively, without detecting a presence of Gram-negative bacteria or component(s) thereof, e.g., administered to an immunosuppressed patient. In one embodiment, a patient's body develops a sepsis in response to the presence of Gram-negative bacteria or component(s) thereof, preferably lipopolysaccharide, in the bloodstream of the patient. In one embodiment, Gram-negative bacteria causing sepsis are *E. coli, Haemophilus* spp., *Klebsiella* spp., *Morganella morganii*, *Citrobacter* spp., *Enterobacter* spp., *Proteus* spp., *Serratia* spp., *Pseudomonas* spp., *Flavobacterium* spp*.*,*Acinetobacter* spp., *Stenotrophomonas maltophilia, Legionella* spp., *Capnocytopha* spp. or *Neisseria meningitidis.* In one embodiment, Gram-negative bacteria causing sepsis are Enterobacterales. In one embodiment, Gram-negative bacteria causing sepsis are resistant to Penicillins, Cephalosporins Carbapenems, Fluoroquinolones, Colistin or Tigecycline. In one embodiment, Gram-negative bacteria are multidrug-resistant (MDR).

In one embodiment, the term "non-human BPI or a fragment thereof', as used herein, relates to BPI or a fragment thereof which is not human BPI or a fragment thereof, e.g., BPI of a Deuterostomia species or a fragment thereof such as BPI of a Neopterygii species or a fragment thereof. In one embodiment, said non-human BPI or a fragment thereof is BPI of a species other than the human species, such as BPI of a Deuterostomia species, such as BPI of a fish species, an amphibian species, a reptile species, a bird species, or a mammalian species other than human; preferably is BPI of a Deuterostomia species; more preferably is non-human vertebrate BPI; even more preferably is teleost BPI. In one embodiment, human BPI has a sequence as defined in SEQ ID No. 24. In one embodiment, said non-human BPI comprises or consists of a sequence having any of SEQ ID No. 1, 26-34, preferably SEQ ID No. 1. In one embodiment, said non-human BPI is teleost BPI having an amino acid sequence of SEQ ID No. 1, 26, and 29-34, preferably SEQ ID No. 1. In one embodiment, said non-human BPI or a fragment thereof comprises an amino acid sequence of SEQ ID No. 1, an N-terminal fragment of BPI having an amino acid sequence of SEQ ID No. 2, and/or an amino acid sequence of any of SEQ ID No. 3-18. In one embodiment, when referring to BPI "of a species", such expression is meant to be understood as comprising BPI obtained, e.g., extracted, from an organism of said species, and/or BPI produced using an amino acid sequence and/or nucleic acid sequence, or fragment(s) thereof, encoding BPI or fragment(s) thereof, of said species. In one embodiment, BPI of a Deuterostomia species or a fragment thereof is BPI of an Actinopterygii species or a fragment thereof. In one embodiment, a Deuterostomia species is preferably an Actinopterygii species, more preferably a Neopterygii species.

In one embodiment, the term "fragment", as used herein, relates to fragment of BPI having an LPS-neutralizing function and/or a function as set forth in any of the foregoing or following paragraphs, such as a bactericidal/permeability-increasing function, an LPS-neutralizing function, and/or bactericidal function; preferably having an LPS-neutralizing function. In a preferred embodiment, the fragment is a biologically active fragment maintaining the biological function of BPI, particularly the bactericidal/permeability-increasing function, the LPS-neutralizing function, and/or the bactericidal function. In one embodiment, the fragment alone or in combination with further fragments of BPI, facilitates a bactericidal/permeability-increasing function, an LPS-neutralizing function, and/or a bactericidal function; preferably an LPS-neutralizing function. In one embodiment, when referring to a fragment "having" a function, such fragment has the function alone or in combination with further BPI fragments, e.g., has such function when being part of a functional protein confirmation in combination with said further fragments, e.g., when being part of an LPS-binding site together with other BPI fragments. In one embodiment, when referring to BPI or a fragment thereof "having" a sequence, e.g., a sequence of SEQ ID No. 1, such expression is meant to be understood as said BPI or fragment thereof comprising or consisting of said sequence. In one embodiment, the terms "having a sequence" and "comprising a sequence" are used interchangeably.

In one embodiment, BPI and/or one or more fragments of BPI are incorporated into a protein scaffold or fusion protein and is/are administered in the form of a protein scaffold or fusion protein comprising BPI and/or one or more BPI fragments. In one embodiment, said BPI or fragment thereof, and/or said protein scaffold or fusion protein comprising BPI and/or one or more BPI fragments, is/are administered to a patient in need thereof in the form of a protein or in the form of a nucleic acid encoding said BPI or fragment thereof and/or said protein scaffold or fusion protein.

The term "non-human vertebrate BPI", as used herein, relates to BPI of vertebrate species other than human species, e.g., BPI of an Actinopterygii species such as rockfish BPI. In one embodiment, non-human vertebrate BPI is BPI of any species selected from fish species such as rockfish species, amphibian species, reptile species, bird species, and mammalian species other than human species, preferably selected from Actinopterygii species such as gaBPI, icBPI, saBPI1, saBPI2, cyBPI, sciBPI, scoBPI, paBPI, leBPI, acBPI, and any combination thereof, preferably having any of SEQ ID No. 1, 26-34. In a preferred embodiment, non-human vertebrate BPI is BPI of a ray-finned fish, preferably teleost BPI. The term "teleost BPI", as used herein, relates to BPI of a teleost such as rockfish BPI, e.g., BPI of *Sebastes schlegelii*, preferably BPI comprising or consisting of a sequence as defined in SEQ ID No. 1. In one embodiment, teleost BPI is selected from gaBPI, icBPI, saBPI1, saBPI2, cyBPI, sciBPI, scoBPI, paBPI, and any combination thereof, preferably having any of SEQ ID No. 1, 26, and 29-34. In one embodiment, teleost BPI is not paBPI. Teleosts are a large infraclass in the class Actinopterygii which are the ray-finned fishes. In one embodiment, BPI, preferably teleost BPI, is BPI of a species selected from rockfish, preferably *Sebastinae* sp., more preferably BPI of a *Sebastes* sp., even more preferably BPI of *Sebastes schlegelii.* In one embodiment, said BPI is non-human vertebrate BPI or a fragment thereof, preferably teleost BPI or a fragment thereof, more preferably is BPI of *Sebastes schlegelii* or a fragment thereof. The term "BPI", as used herein, is meant to be understood as relating to BPI and fragments of BPI, preferably to BPI and biologically active fragments of BPI having an LPS-neutralizing function. In one embodiment, said BPI, particularly non-human vertebrate BPI, has a sequence selected from SEQ ID No. 1-7 or 26-34. In one embodiment, said BPI, preferably teleost BPI, more preferably teleost BPI having a sequence of SEQ ID No. 1-7, 26, and 29-34, even more preferably SEQ ID No. 1, comprises a modification selected from
- an amino acid substitution at position 347 of SEQ ID No. 1, preferably an asparagine at position 347 being substituted with alanine;
- one or more amino acid substitutions providing a sequence of any of SEQ ID No. 19-23;
- a modified glycosylation pattern, preferably a removed glycosylation e.g., at position 347 of SEQ ID No. 1; wherein, optionally, said glycosylation pattern is modified by a mild acid treatment, an amino acid substitution, and/or an enzymatic treatment, preferably a glucosidase treatment e.g., using mannosidase;
and a combination thereof.

In one embodiment, a "modification" is an amino acid substitution, preferably an amino acid substitution of at least one amino acid of a sequence of BPI, preferably BPI having a sequence of SEQ ID No. 1, e.g., an amino acid substitution providing a sequence of any of SEQ ID No. 19-23 or an amino acid substitution removing a glycosylation site, e.g., an amino acid substitution of an asparagine with an alanine or aspartic acid such as an asparagine at position 347 of SEQ ID No. 1 being substituted with alanine. For example, if a glycosylation site, such as position 347 of SEQ ID No. 1, is modified by an amino acid substitution, e.g., an exchange of a glycosylation site asparagine by a different amino acid such as aspartic acid or alanine, such glycosylation site can no longer be glycosylated, and the glycosylation pattern is thus modified, particularly the glycosylation is removed. In one embodiment, said BPI comprising a modification has a sequence identity with a sequence of SEQ ID No. 1-7 or 26-34 of at least 80%, preferably 90%, more preferably 95%, even more preferably 99%.

In one embodiment, when referring to one or more amino acid substitutions "providing" a sequence of any of SEQ ID No. 19-23, such term is meant to be understood as relating to a scenario in which one or more amino acids of a BPI amino acid sequence, e.g., BPI having a sequence of any of SEQ ID No. 1-7, 26-34, is/are substituted (e.g., by point mutation) and/or incorporated to obtain a sequence of interest within said BPI amino acid sequence such as a sequence as defined in any of SEQ ID No. 19-23 within said BPI amino acid sequence. For example, by such one or more amino acid substitutions providing a particular sequence e.g., of any of SEQ ID No. 19-23, the amino acid substitutions result in a BPI sequence, e.g., a sequence having at least 80% or 90% sequence identity with any of SEQ ID No. 1-7, 26-34, comprising said particular sequence e.g., of any of SEQ ID No. 19-23. A BPI sequence, e.g., a sequence having any of SEQ ID No. 1-7, 26-34 can be modified by incorporating a sequence as defined in any of SEQ ID No. 19-23 and/or by substituting one or more amino acids to obtain a sequence as defined in any of SEQ ID No. 19-23 within said BPI sequence.

By such amino acid substitution(s), a sequence of interest, such as an LPS-binding motif or glycosaminoglycan-binding motif, can be incorporated into said BPI amino acid sequence. In one embodiment, by such amino acid substitution providing a sequence of interest within said BPI amino acid sequence, such as an LPS-binding motif or glycosaminoglycan binding motif (e.g., any of SEQ ID No. 19-23), the function of BPI is even further enhanced, for example an LPS-neutralizing function is even further enhanced compared to non-modified BPI. In one embodiment, LPS-binding motifs and glycosaminoglycan binding motifs overlap in their function to neutralize LPS. In one embodiment, one or more amino acid substitutions provide a sequence of any of SEQ ID No. 19-23 within an amino acid sequence of BPI, preferably within an amino acid sequence of BPI having SEQ ID No. 1, more preferably within an amino acid sequence of SEQ ID No. 1 having any of SEQ ID No. 2-7.

In one embodiment, a modified glycosylation pattern is a removed glycosylation pattern, e.g., a modified BPI not having any glycosylation. In one embodiment, the modified glycosylation pattern comprises or consists of a removed glycosylation site, preferably a removed glycosylation site at position 347 of SEQ ID No. 1, e.g., by amino acid substitution of a glycosylation site such as an asparagine to an amino acid other than said asparagine. In one embodiment, the modified glycosylation pattern comprises a consists of removed glycosylation caused by a mild acid treatment, an amino acid substitution, and/or an enzymatic treatment.

In one embodiment, said BPI neutralizes lipopolysaccharide. In one embodiment, said BPI alleviates said infection and/or said sepsis by neutralizing a lipopolysaccharide of said Gram-negative bacteria. The non-human BPI or fragment thereof for use of the present invention is highly effective in neutralizing lipopolysaccharide. Surprisingly, non-human BPI, such as teleost BPI, has a higher LPS-neutralizing efficiency than human BPI. Thus, unexpectedly, non-human BPI, such as teleost BPI, is a highly effective means for alleviating a Gram-negative infection and an associated sepsis by neutralizing a lipopolysaccharide of said Gram-negative bacteria. In one embodiment, the term "neutralizing lipopolysaccharide", as used herein, relates to a binding to lipopolysaccharide that decreases and/or inhibits the biological activity of LPS and/or the immunostimulatory potential of LPS.

The term "N-terminal fragment of BPI", as used herein, relates to a fragment of BPI comprising the N-terminus of BPI, e.g., a fragment comprising the N-terminal 193 amino acids or a fragment comprising the N-terminal 199 amino acids of BPI, such as a BPI fragment comprising a consisting of an amino acid sequence of SEQ ID No. 2. In one embodiment, a N-terminal fragment is analogous to a BPI fragment as described in [3].

In one embodiment, said BPI for use is administered to a patient in need thereof; preferably administered systemically or locally; more preferably administered intravenously, intravascularly, orally, nasally, mucosally, intrabronchially, intrapulmonarily, intradermally, subcutaneously, intramuscularly, intravascularly, intraperitoneally, intrathecally, intracerebral, intracranial, intraocularly, intraarticularly, intranodally, intratumorally, and/or intrametastatically. In one embodiment, said BPI is administered in the form of a protein and/or nucleic acid. In one embodiment, said BPI for use is administered in the form of a protein, in the form of DNA, or in the form of mRNA. In one embodiment, said BPI is a full-length protein or a fragment thereof. In one embodiment, if said BPI for use is administered in the form of a nucleic acid, e.g., in the form of DNA or in the form of mRNA, the nucleic acid comprises a nucleic acid sequence encoding a signal peptide, preferably a nucleic acid sequence encoding a signal peptide as defined by amino acids 1-31 of SEQ ID No. 25.

The term "patient", as used herein, relates to a human or an animal. In one embodiment, said patient is a newborn, a toddler, or a patient having an age of at least 40 years, preferably of at least 60 years. In one embodiment, said patient is immunocompromised. In one embodiment, said patient is characterized by BPI deficiency, e.g., a decreased BPI level and/or a functional BPI deficit; neutropenia; defective neutrophil function; and/or by increased levels of anti-neutrophil cytoplasmic antibodies, preferably anti-BPI autoantibodies. Surprisingly, non-human BPI is particularly useful in treating patients having increased levels of anti-neutrophil cytoplasmic antibodies such as anti-BPI antibodies, since non-human BPI, e.g., scoBPI, is not neutralized and/or bound by said antibodies. In one embodiment, a patient having increased levels of anti-neutrophil cytoplasmic antibodies such as anti-BPI antibodies is a patient having a disease selected from infections, e.g., HIV infections, Gram-positive or Gram-negative bacteremia; autoimmune diseases e.g., systemic lupus erythematodes, psoriasis, rheumatic diseases such as rheumatoid arthritis, granulomatosis with polyangiitis, and inflammatory bowel diseases such as Crohn's disease and ulcerative colitis; hematologic diseases; cystic fibrosis; COPD; alpha-1 antitrypsin deficiency; bronchiectasis; TAP deficiency; primary biliary cirrhosis; vasculitis, e.g., autoimmune vasculitis; preferably SLE. In one embodiment, said "increased" levels of anti-neutrophil cytoplasmic antibodies are increased in a patient compared to a healthy individual. In one embodiment, BPI deficiency comprises BPI levels, e.g., BPI protein levels, being decreased in a patient compared to a healthy individual, and/or the function of BPI being decreased in a patient compared to a healthy individual. In one embodiment, said patient has increased levels of anti-neutrophil cytoplasmic antibodies, preferably anti-BPI autoantibodies, compared to a healthy individual. In one embodiment, BPI for use of the invention is for use in preventing or treating a sepsis associated with an infection with Gram-negative bacteria in a patient characterized by having increased levels of anti-neutrophil cytoplasmic antibodies such as anti-BPI antibodies, preferably a patient having a disease selected from infections, e.g., HIV infections, Gram-positive or Gram-negative bacteremia; autoimmune diseases e.g., systemic lupus erythematodes, psoriasis, rheumatic diseases such as rheumatoid arthritis, granulomatosis with polyangiitis, and inflammatory bowel diseases such as Crohn's disease and ulcerative colitis; hematologic diseases; cystic fibrosis; COPD; alpha-1 antitrypsin deficiency; bronchiectasis; TAP deficiency; primary biliary cirrhosis; vasculitis, e.g., autoimmune vasculitis, more preferably an SLE patient. It has been demonstrated that anti-neutrophil cytoplasmic antibodies such as anti-BPI autoantibodies compromise the function of BPI. The inventors have shown that non-human BPI is not recognized by autoantibodies, such as scoBPI, and that substitution of hoBPI with a non-human BPI is highly effective in the treatment of sepsis by neutralizing LPS as shown by reduction of pro-inflammatory cytokines as exemplified for IL-6. In one embodiment, a functional BPI deficit comprises the patient being characterized by a single nucleotide polymorphism (SNP) associated with an increased risk of Gram-negative infections, of Gram-negative sepsis or a negative outcome thereof, such as a SNP at position rs4358188 (AA185 in SEQ ID No. 24) or at position rS1341023 (AA12 in SEQ ID No. 25) on at least one allele.

In one embodiment, said patient suffers from or is at risk of acquiring, in addition to said infection with Gram-negative bacteria, a disease selected from cancer; neutropenia; infections other than said infection with Gram-negative bacteria, e.g., HIV infections; autoimmune diseases, e.g., systemic lupus erythematodes, psoriasis, rheumatic diseases such as rheumatoid arthritis, granulomatosis with polyangiitis, and inflammatory bowel diseases such as Crohn's disease and ulcerative colitis; hematologic diseases; cystic fibrosis; COPD; alpha-1 antitrypsin deficiency; bronchiectasis; TAP deficiency; primary biliary cirrhosis; vasculitis; and immune suppression, e.g., iatrogenic immune suppression and therapeutic immune suppression. In one embodiment, an autoimmune disease with special benefit from administration of non-human BPI, such as teleost BPI including scoBPI, is a disease associated with increased levels of anti-neutrophil cytoplasmic antibodies, preferably anti-BPI autoantibodies, such as SLE.

The term "coadministration", as used herein, refers to combined administration of BPI with other substances, such as drugs, e.g., an antimicrobial agent, an anti-inflammatory agent, an immunosuppressive agent, or an immunotherapeutic agent. In one embodiment, a coadministration of BPI and at least one other substance, e.g., an antimicrobial agent, an anti-inflammatory agent, an immunosuppressive agent, or an immunotherapeutic agent, is carried out simultaneously or consecutively. In one embodiment, said BPI or fragment thereof, e.g., in the form of a protein or a nucleic acid, is co-administered with any of
- an antimicrobial agent, preferably selected from antibacterial agents, antifungal agents, virostatic agents, and antiparasitic agents, more preferably an antibiotic;
- an anti-inflammatory agent, preferably selected from steroids, non-steroidal antiphlogisitic agents, and antibodies targeting a pro-inflammatory cytokine or an immune receptor;
- an immunosuppressive agent, preferably selected from cylosporin, tacrolimus, mycophenolat, and methotrexate;
- an immunotherapeutic agent, preferably selected from anti-TNF antibodies, anti-BAFF antibodies, anti-CD20 antibodies, immune checkpoint inhibitors, TLR ligands, and C-type lectin ligands, e.g., glucans;
and combinations thereof.

In one embodiment, said antimicrobial agent is selected from β-lactam antibiotics, including penicillins, carbapenems (such as imipenem or meropenem) and cephalosporins (such as or ceftriaxone), chinolones (such as ciprofloxacin), aminoglycosides, polymyxins (such as polymyxin B), rifamycins (such as rifamycin B), lipiarmycins, sulfonamides, macrolides (such as azithromycin), lincosamides, tetracyclines, cyclic lipopeptides (such as daptomycin), glycylcyclines (such as tigecycline), oxazolidinones (such as linezolid), lipiarmycins (such as fidaxomicin), glycopeptides (such as vancomycin), metronidazole, tuberculostatic drugs, antifungal drugs, and virostatic drugs.

The term "effective amount", as used herein, relates to an amount of BPI or fragment thereof for use in a method of preventing or treating a sepsis associated with an infection with Gram-negative bacteria, said amount having an LPS-neutralizing effect. In one embodiment, said amount is an amount that alleviates said sepsis, preferably by neutralizing LPS present in a patient's body due to an infection with Gram-negative bacteria. In one embodiment, an effective amount of BPI is in the range of from 1 pg/kg/dose to 1 g/kg/dose, preferably 100 pg/kg/dose to 300 mg/kg/dose, more preferably 1 ng/kg/dose to 30 mg/kg/dose, even more preferably 0.5 mg/kg/dose to 20 mg/kg/dose, wherein kg relates to the body weight of the patient. In one embodiment, the dose is administered once per day or is split into several administrations per day.

In one embodiment, said fragment of BPI comprises at least one of a N-terminal lipophilic binding pocket and a lipopolysaccharide binding site, or combinations thereof. In one embodiment, the N-terminal lipophilic binding pocket is as described in [4]. In one embodiment, said lipopolysaccharide binding site is a lipopolysaccharide binding site as defined in [3] or a homologue or orthologue thereof, e.g., a sequence having at least 20%, preferably 30% sequence identity with a lipopolysaccharide binding site as defined in [3]. In one embodiment, regions I-III having a sequence as defined in SEQ ID No. 5-7, are derived from the teleost orthologue as defined in SEQ ID No. 1 and correspond to the human BPI regions I-III as defined in [3]. In one embodiment, non-human BPI, preferably non-human vertebrate BPI, more preferably teleost BPI, comprises at least 5, preferably at least 6, more preferably at least 7, even more preferably at least 8, 9, or 10, amino acids selected from arginine, histidine, and lysine. In one embodiment, any of regions I-III of non-human BPI, preferably of non-human vertebrate BPI, more preferably of teleost BPI, comprise(s) at least 4, preferably at least 5, amino acids selected from arginine, histidine, and lysine. Without wishing to be bound by any theory, the inventors believe that teleost BPI has a high number of positively charged amino acids exposed by the three-dimensional conformation of BPI, such exposure of a high number of positively charged amino acids enhancing the LPS-neutralizing efficiency of teleost BPI compared to human BPI. Furthermore, teleost BPI, e.g., scoBPI, has a high amount of positively charged amino acids in regions I, II, III, A, and B which contributes to the LPS-neutralizing efficiency of teleost BPI. BPI for use of the invention has the advantage that, surprisingly, it has a higher LPS-neutralizing efficiency compared to human BPI. By neutralizing LPS with high efficiency, BPI for use of the invention is highly effective in preventing or treating sepsis associated with infections with Gram-negative bacteria. Particularly, BPI for use of the invention neutralizes LPS with high efficiency and thus reduces the amount of LPS causing an immune reaction within the body system of the patient. BPI for use of the invention is particularly useful in the prevention or treatment of Gram-negative sepsis in patients characterized by increased levels of anti-neutrophil cytoplasmic antibodies, e.g., anti-BPI autoantibodies, as non-human BPI is not neutralized by said antibodies. It has been observed that the functionality of hoBPI is inactivated by anti-neutrophil cytoplasmic antibodies such as anti-BPI autoantibodies. Advantageously, non-human BPI is not bound by anti-neutrophil cytoplasmic antibodies such as anti-BPI autoantibodies, so that non-human BPI is highly effective in the treatment of patients characterized by increased levels of anti-neutrophil cytoplasmic antibodies, e.g., anti-BPI autoantibodies.

In one embodiment, teleost BPI comprises or consists of a sequence of SEQ ID No. 1-7, 26 and 29-34. In one embodiment, BPI is teleost BPI and comprises or consists of a sequence of SEQ ID No. 1-7, 26 and 29-34. In one embodiment, Actinopterygii BPI, i.e., BPI of an Actinopterygii species, comprises or consists of a sequence of SEQ ID No. 1-7, 26-34. In one embodiment, BPI is Actinopterygii BPI and comprises or consists of a sequence of SEQ ID No. 1-7, 26-34. In one embodiment, Neopterygii BPI, i.e., BPI of an Neopterygii species, comprises or consists of a sequence of SEQ ID No. 1-7, 26 and 28-34. In one embodiment, BPI is Neopterygii BPI and comprises or consists of a sequence of SEQ ID No. 1-7, 26 and 28-34. In one embodiment, non-human vertebrate BPI comprises or consists of a sequence of SEQ ID No. 1-7, 26-35. In one embodiment, BPI is non-human vertebrate BPI and comprises or consists of a sequence of SEQ ID No. 1-7, 26-35. In one embodiment, non-human BPI comprises or consists of a sequence of SEQ ID No. 1-7, 26-36. In one embodiment, BPI is non-human BPI and comprises or consists of a sequence of SEQ ID No. 1-7, 26-36. In one embodiment, BPI for use of the invention comprises or consists of a sequence of any of SEQ ID No. 1-7, 26-34, preferably SEQ ID No. 1-7, 26 and 28-34, more preferably SEQ ID No. 1-7, 26 and 29-34, even more preferably SEQ ID No. 1-7.

### Sequences

SEQ ID No. 1: complete sequence (AA 1-455) of scoBPI 
INPGVKVRLTGKGLEYGRQLGMASIQQKLKTIKVPDISGKQRVSPIGKVKYSLSNMQIVDVGL
PKSALDLVPGTGVKLSIGNAFLRMHGNWRVKYLRIIKDSGSFDLNVNDLTITTTIAIKSDETG
RPVVSSVNCAATVGSAKIKFHGGASWLYNLFRKFVDKAIRNALQKQMCPLVANAVSDLNPQ
LKTLNVLAKVDQYAEIEYSMVSSPTVSNSCIDFSLKGEFYNIGKHQEPPFSPAAFSLPPQINN
MLYISVSAFTINSAAFVYNTAGALSLYITDDMIPQASPIRLNTRTFGAFIPQVAKRFPGLMMKL
LVKTAKNPVVTFEPNKVTVQATSTVTAYAIQPNTTLSPLFVLNLETSVSARVFVSGMRLAGAV
 
TLNKMALTLGTSYVGEFQVRSLDSIFQVVLKVVVIPILNVQLAKGYPLPTLGKMKLVNTELQV
LKDYMLIGTDVQFTG
 SEQ ID No. 2: N-terminus (AA 1-199) of scoBPI 
INPGVKVRLTGKGLEYGRQLGMASIQQKLKTIKVPDISGKQRVSPIGKVKYSLSNMQIVDVGL
PKSALDLVPGTGVKLSIGNAFLRMHGNWRVKYLRIIKDSGSFDLNVNDLTITTTIAIKSDETG
RPVVSSVNCAATVGSAKIKFHGGASWLYNLFRKFVDKAIRNALQKQMCPLVANAVSDLNPQ
LKTLNVLAKVDQ
 SEQ ID No. 3: region A (partial sequence of scoBPI)

INPGVKVRLTGKGLEYGRQLGMASIQQKLKTIKVPDISGKQRVSPIGKVKYSLSNMQIVDVGL
PKSALDLVPGTGVKLSIGNAFLRMHGNWRVKYLR

| | |
|---|---|
| SEQ ID No. 4: | region B (partial sequence of scoBPI) AKIKFHGGASWLYNLFRKFVDKAIRNALQKQMCPLVAN |
| SEQ ID No. 5: | region I (partial sequence of scoBPI) GRQLGMASIQQKLKTIKVPDISGKQRVSPIGKVK |
| SEQ ID No. 6: | region II (partial sequence of scoBPI) DVGLPKSALDLVPGTGVKLSIGNAFLRMHGNWRVKYLRIIK |
| SEQ ID No. 7: | region III (partial sequence of scoBPI) AKIKFHGGASWLYNLFRKFVDKAIRNA |
| SEQ ID No. 8: | sequence of interest (partial sequence of scoBPI) VKVRL |
| SEQ ID No. 9: | sequence of interest (partial sequence of scoBPI) QKLKT |
| SEQ ID No. 10: | sequence of interest (partial sequence of scoBPI) GKQRV |
| SEQ ID No. 11: | sequence of interest (partial sequence of scoBPI) GKVKY |
| SEQ ID No. 12: | sequence of interest (partial sequence of scoBPI) LRMHG |
| SEQ ID No. 13: | sequence of interest (partial sequence of scoBPI) WRVKYLRIIKD |
| SEQ ID No. 14: | sequence of interest (partial sequence of scoBPI) AKIKFHG |
| SEQ ID No. 15: | sequence of interest (partial sequence of scoBPI) KAIRN |
| SEQ ID No. 16: | sequence of interest (partial sequence of scoBPI) IRLNTR |
| SEQ ID No. 17: | sequence of interest (partial sequence of scoBPI) |
| | MKLLVKT |
| SEQ ID No. 18: | sequence of interest (partial sequence of scoBPI) GKMKL |
| SEQ ID No. 19: | LPS binding motif, wherein B is a basic amino acid and X is any amino acid XBBXBX |
| SEQ ID No. 20: | LPS binding motif, wherein B is a basic amino acid and X is any amino acid XBBXXBX |
| SEQ ID No. 21: | glycosaminoglycan binding motif, wherein B is a basic amino acid and X is any amino acid XBXBX |
| SEQ ID No. 22: | glycosaminoglycan binding motif, wherein B is a basic amino acid and X is any amino acid XBXXBX |
| SEQ ID No. 23: | glycosaminoglycan binding motif, wherein B is a basic amino acid and X is any amino acid XBXXXBX |

SEQ ID No. 24: complete sequence (AA 1-456) of human BPI 
VNPGVVVRISQKGLDYASQQGTAALQKELKRIKIPDYSDSFKIKHLGKGHYSFYSMDIREFQL
PSSQISMVPNVGLKFSISNANIKISGKWKAQKRFLKMSGNFDLSIEGMSISADLKLGSNPTSG
KPTITCSSCSSHINSVHVHISKSKVGWLIQLFHKKIESALRNKMNSQVCEKVTNSVSSELQPY
FQTLPVMTKIDSVAGINYGLVAPPATTAETLDVQMKGEFYSENHHNPPPFAPPVMEFPAAH
DRMVYLGLSDYFFNTAGLVYQEAGVLKMTLRDDMIPKESKFRLTTKFFGTFLPEVAKKFPN
MKIQIHVSASTPPHLSVQPTGLTFYPAVDVQAFAVLPNSSLASLFLIGMHTTGSMEVSAESNR
LVGELKLDRLLLELKHSNIGPFPVELLQDIMNYIVPILVLPRVNEKLQKGFPLPTPARVQLYN
VVLQPHQNFLLFGADVVYK
 SEQ ID No. 25: complete sequence (AA 1-487) of human BPI with signal peptide 
MRENMARGPCNAPRWASLMVLVAIGTAVTAAVNPGVVVRISQKGLDYASQQGTAALQKEL
KRIKIPDYSDSFKIKHLGKGHYSFYSMDIREFQLPSSQISMVPNVGLKFSISNANIKISGKWKA
QKRFLKMSGNFDLSIEGMSISADLKLGSNPTSGKPTITCSSCSSHINSVHVHISKSKVGWLIQL
FHKKIESALRNKMNSQVCEKVTNSVSSELQPYFQTLPVMTKIDSVAGINYGLVAPPATTAETL
DVQMKGEFYSENHHNPPPFAPPVMEFPAAHDRMVYLGLSDYFFNTAGLVYQEAGVLKMTL
RDDMIPKESKFRLTTKFFGTFLPEVAKKFPNMKIQIHVSASTPPHLSVQPTGLTFYPAVDVQA
FAVLPNSSLASLFLIGMHTTGSMEVSAESNRLVGELKLDRLLLELKHSNIGPFPVELLQDIMN
YIVPILVLPRVNEKLQKGFPLPTPARVQLYNVVLQPHQNFLLFGADVVYK
 SEQ ID No. 26: complete sequence (AA 1-455) of gaBPI 
ANAGVKVKLTQKGLDFGRELAVASLQKKLMTIKVPDISGKERVKPIGKVRYSVTEMRVVKLG
LPSSAIGLVPGTGFSLTITNAFLSLHGRWRVKYLRFIKDRGSFDLAIKSLSITTSISLRSDNMGL
PAVAMASCTTTLGGVSVKLHGGASWLYNLFRRFIEKGLQSQLQKKLCPLVAESVSSMNQFLK
TVNVMAPVDRYAEIAYPMVSSPDISSSSIGLNLKGEFYNIGNHMEPPFSPAPFFLPNQEQSML
YIGLSAFTANSASFVYNKAGTLSLKITDDMVPRSSPIRLTTNTFGVLIPQIAKLFPGLMMVLQL
KMTNDPLSSIEANKVTVDAKSSLTAYAILANTKLAPLFVLNLDFSVSARVNVVGNKLAGAVTL
 
NRLDMSLGKSYVGDFKVGSLSNIFQIVLKWVVLPTVNVRLKEGFPLPSLGKINLKNTQLQILK
GYIMIGTDLQNSG
 SEQ ID No. 27: complete sequence (AA 1-461) of acBPI 
TNPGITGRITQNGLEYGRQIGMAVLQQKLKSIKIPDMSGKEKVSPIGKVRYSLTEMQIESVSL
PKSAVGLSPGTGVVLSIGNAYISIKGNWRVKYRRIVKDHGSFDLSVSGLTISAGIGVSRDETGR
PTVSSASCSTSIGGVRIKFHGGASWLYNLFSKFIEKALRKALSDKICPLVSDAIGGLNPHLKTL
NVLAKVDRYAEVEYSLVSPPVITKNYIDLDLKGEFYNIGQHHEPPFTAPPFSLPEQASSMLYIG
VSEFTLNSAGFVYYSAGVLNTNVTDDMIAQSYPNMSVKLIVQATQQPHATMQPSNVTVEVM
GSITAYVIKPDSTLAPLFILGMKASTSANLLISGKKLVGSVTLNGLVLSLQKSYVGPFQIQSLQT
VLQLALKSAVLPKVNELLKEGFPLPTIDKMNLVNAQIQILKDYVLIGTDVAFTGSPARYSDTA
KARYLSSVYYHYYTCLT
 SEQ ID No. 28: complete sequence (AA 1-455) of leBPI 
TNPGITVRLTQKGLEYGRQIGIAVLQQKLKSVHIPDISGKESVSPVGSVEYSLSGMQVVSLGLP
QSSLVLAPGSGLSLSIGNAYISIDGNWRVKYLKFIKDHGSFDLSVSELTISAVMAVGSDETGRP
TVSCTSCSASVGSAKIKFHGGASWLYNLFSSFIDKALRNALQDKLCPLVTDAIAGINPHLKTL
NVLAKVDQYAEIEYSMVSSPVITKDYIDLNLKGEFYNIGKHQEPPFPAPGFSLPAQANSMLYF
GVSQFTPNSAGFVYSTTGVLSINVTDDMIPKSSPLRLNTKTFGAFIPEIAKRYPGLQMELLVKS
TKQPFVTFQTNNMTLQAVGSIAAYAIQPNSTLTPLFILSVDASVSGNVYVSGVKVAGSLSLNSF
KLSLVTSYVGPFQVQSLSNIFQLVLKVAVLPKVNELLQQGFPLPTIEKMNLVNTQLQIQKDYL
LIGTDVQFSP
 SEQ ID No. 29: complete sequence (AA 1-455) of icBPI 
TNPGVILRVTQKGLEYGRQIGLVTLQKKLKTVKIPDMSGSEKVSPIGKVSYSLTGIQILDLGLP
KSAVGLVPGTGVSLSIGDAYINLHGNWRVKYLRIIKDSGSFDLSVSGLSISATISVKGDDTGRP
VVSSANCAASVGNVKIKFHGGASWLYNLFKHYIEKALRSELQKQICSLVAEAIEEMNPHLKT
LNVLAQVDKYAEIEYSMVESPLMSNSSIDLSLKGEFYNIGQHKEPPFSPTPFSPPAQDSNMM
YIGLSAFTLNSAGFVYNNAGVLSLYITDDMIPSSFPIRLNTKTFGTFIPQIAKQYPGLMMKLLV
KAEKEPNVSFEPDNVTLQASSTVTAYAIQPNATLSPLFVLNVNASVSARIYVSGLTVAGNLSL
DEMDMTLATSYVGPFQVKSLDSFLTMIMKAVVIPVVNALLRQGYPLPAIGNMNLVNTQLQIL
KDYVLIGTDVQFTG
 SEQ ID No. 30: complete sequence (AA 1-455) of saBPI1 
ANPGVKVKLTDKGIEYGKQIGMASLQQKLKTIKVPDLSGTEKVPHVGKVKYSLTGMTIVNLG
LPKSALVLVPGTGVSLAITNAFINLHGNWRVRYFRFMQDRGSFDLAVNGLTITANIAIKSDET
GRPMVSTVNCVANVGRASIKFHGGASWLYNLFKSYIDKALRSALQKQICPLVADAITDMNQ
HLKTFNVLAKVDQYAEIEYSMGTSPTISKSSMEFSLKGEFYNIVKHQEPPFSPTPFSLPPQGN
NMLYIGVSSFTPNSAGFVYNNAGALSLYVTDDMIPPSSPIRLNTGTFGVFIPEIAKRFPGMMM
KLLVKTVKEPTISLEPNNVTVQASGTVTAYAIQPNTTLSPLFVLNMEGSVSAQLNVTGVKLAG
AITLNKIEMTLGTSYVGQFQVQSLDNIFLMVLKVVVIPKVNARLEKGFPLPSIGKMNLVNTQL
QVLKDYMLIGTDVQFTG
 SEQ ID No. 31: complete sequence (AA 1-455) of saBPI2 
ASPGVKVKLTDKGIEYGKQIGMASLQQKLKTMKVPDLSGTEKVPPIGKVKYSLTGMTIVNLG
LPKSALVLMPGTGVRLAITNAFINLHGNWRVRYFRFIQDRGSFDLAVNGLTITADIAIKSDET
GRPTVSTVNCVANVGSASIKFHGGASWLYNLFKSYIDKALRSALQKQICPLVADVITDMNPH
LKTFNVLAKVDQYAEIEYSMVTSPTISKSSIEFSLKGEFYNIGKHQEPPFSPTPFSLPPQDNNM
LYIGVSSFTPNSAGFVYNNAGALSLYVTDDMIPPSSPIRLNTGTFGVFIPEIAKRFPGMMMKL
LVKTVKEPTISLEPNNVTVQASGTVTAYAIQPNTTLSPLFVLNMEGSVSAQMNVTGVKLAGAI
 
TLNKIEMTLGTSYVGQFQVQSLDNIFLMVLKVVVIPKVNARLEKGFPLPTIGKMNLINTQLQ
VLKDYMLIGTDVQFTG
 SEQ ID No. 32: complete sequence (AA 1-455) of cyBPI 
TNPGVKVRLTQKGLEYGRQLGISSIQQKLRTIKVPDISGKQKVSPIGKVEYSLSNMNIVDVGL
PKSAVTPVAGTGIKLSIGDAFIHLHGNWRVKYLRVIKDSGSFDLSVNALAISTSIAIHSDETGR
PTVSSVSCEAHVGSVSVKFHGGASWLYNLFTNFIDKALRNTLEKQICPLVADAVSDMNPHLK
TLNVIAKVDKYTEIEYSMVTSPTVSESSIDLSLKGEFYSIGKHQEAPFSPVAFFLPPQANNMLY
FGISAYTPNTAAFAYNSAGALSLYITDDMIPQSSPIRLNTKTFGTFIPQIAKRYPGLMMKLLLQ
TIKTPNISFQPNNVTIQASATVTAYAIQPNTTLTPLFVLNLNTSVSGKMFVSEMRLAGAVTLN
EMDLTLRTSYIGDFQVRSLDNILQLVLKVVVIPKLNVKLSQGYPLPAIGKMKLVNTQVQVLK
DYMLVGTDVQFTG
 SEQ ID No. 33: complete sequence (AA 1-455) of sciBPI 
INPGVKIELTRKGLEYGRQLGMVSIQRKLKTIKIPDFSGSQSVAPIGKVQYSLSNMQIDNVQL
PQSALDLVPGSGVRLSISNAFISLHGNWRVKYLKIIKDSGTFDLKVSGLSITTTIATKTDETGR
PVVSTVNCSATVNSATIKFHHGASWLYNLFKKFVSKALRQAMEKKMCPLVANAVNDMNPY
LKTLNVVAKVDQYAEIEYSMVSSPTIANSSVDLSLKGEFYNIGKHQEPPFSPTAFSMPPEVNN
MLYIGLSAFTTNSAAFVYNKAGALSLYITDDMLPKSSPIRLNTRTFGVVIPQIAKLFPGLMMK
LLVKTVKTPVITFEPSNVTVQATASVTAYAIQSNGTLTPLFVLNLDASVSAQVFVSGMRLAGA
VTLNNMELTLGTSYVGEFQVKALHSIFQMVLKVVVIPKLNAQLAKGYPLPTLGKMKLVNTQ
LQVLKDYMLIGTDVQFKD
 SEQ ID No. 34: complete sequence (AA 1-455) of paBPI 
TNPGVKVKLTEKGLEYGRQLGMASIQQKLKTIKVPDISGKERVPPIGKVEYSLSHMQIVKLGL
PKSAVDLVPGTGIKLSISNAYISLHGNWRVKYLRIIKDSGSFDLNVNGLTITSSLAIKSDETGR
PTVSSVSCVATVGSVSIKFHGGASWLYNLFKNFIDKALRNALQKQICPLVADAVNELNPHLK
TLNVLAKVDQYAEIEYSMVSSPTVSKSSIDLNLKGEFYNIGKHQEAPFSPTAFSLPPQNDDML
YMGMSAYSVNSAAFVYNKAGALSLYITDDMIPQSSPIRLNTRTFGTFIPEISKRFPGLMMKLL
VQTVKTPVITFQPNNATTQASCTVTAYAIQPNSTLSPLFILNLETSVSAQVFISGMRVAGAVSL
NKMDLTLRTSYVGEFKVRSLDNILQMVLKVVVIPKLNVQLAKGYPLPAIGKMKLVKTQLKVL
KDYMLIGTDVQFTG
 SEQ ID No. 35: complete sequence (AA 1-456) of muBPI 
TDPGFVAMISQKGLDFACQQGVVELQKELQAISVPDFSGVFKIKHLGKGSYEFYSMAVDGFH
IPNPKIEMLPSDGLRVFIKDASIKINGKWMSRKNFLKAGGNFELSIQGVSISTDLILGSDSSGH
ITTICSNCDSHIDSVHIKISGSMLGWLIRLFHRKIETSLKNIIYKKICKIVRDSVSSKLQPYLKTL
SVITRVDDVTSVDYSLLAPLTTTNQFLEGQLKGEFFWRGHRDPLPIHPPVMRFVPNGAYMV
CMGISDYFFNTEVLAYQQSGTLKMTLGGQLLSNNGRFQLNTDFLRTFLPKVAKMFPSMGVQ
LLISAPVPVHLSIQPSGLSFNPKLETQAFVVLPNASLVPLFVLGMKTNASLEVDAEENRLVGE
MKLGSRWLLELKESKFGPFKVEYLEDVINYLVSTLVLPKINERLRRGFPLPLPAGIRFSHFTF
YPYQNFLLLEADLHLI
 SEQ ID No. 36: complete sequence (AA 1-458) of osBPI

KTPGLQTRITDRALEYATEVALDILSKQVTGQQIPDQHGQSGDVKFDITGMNVKQFTKPSSR
VSLIQNVGLSWSTSGTGLAIHGDFKYKYRKGIIKISDHGSFDLKASGINFQVKIEIGMDGTGR
PTMKAVGCSCNVGSADIKFHGGAAWIYSLFSGQLENKLKDMVGGGNGLLCKQLNTLIDVNG
MKSLQKLPVTVQIAKRFLLDYRFLSKPSFQTKFMETYHKGEVYWNAVPVDAPFAAPPLLKSS
DTSRMMYIWLSDYVFNTMSYNALKYNQLQYNVTNKDLPSGVLNTTCPQSTCIGKIIKAIGTK
FPNTTVMLYMKSTSMPNMTAQNGSTVVNASGDIVFFAQQPGGKYTYFLTLSATMSTTISLMI
QNEKVFAKVLKLPISVTVKDSKIPVSPEGLNFIVKGIVSVFVEPKLNELGAAGFPLPVINSVHF
TNTQLTVAKDTLLIATDLKYSG

In one embodiment, a basic amino acid is any of arginine (Arg), lysine (Lys), and histidine (His). In each of the above-mentioned sequences, particularly sequences having any of SEQ ID No. 19-23, B is, at each occurrence, independently selected from basic amino acids comprising arginine, lysine, and histidine, and X is, at each occurrence, independently selected from any amino acid. In one embodiment, in any sequence having any of SEQ ID No. 19-23, B is, at each occurrence, independently selected from basic amino acids, and X is, at each occurrence, independently selected from any amino acids. In one embodiment, a sequence having any of SEQ ID No. 19-23 is preferably located within a sequence having any of SEQ ID No. 2-7, preferably SEQ ID No. 3-7. In one embodiment, BPI comprising a modification comprises a sequence having any of SEQ ID No. 19-23 located within a sequence having any of SEQ ID No. 2-7, preferably SEQ ID No. 3-7.

In one embodiment, SEQ ID No. 3 comprises region A which facilitates LPS-neutralization and bactericidal activity. In one embodiment, SEQ ID No. 4 comprises region B which facilitates LPS-neutralization. In one embodiment, SEQ ID No. 5 comprises region I which facilitates LPS-neutralization. In one embodiment, SEQ ID No. 6 comprises region II which facilitates LPS-neutralization and bactericidal activity. In one embodiment, SEQ ID No. 7 comprises region III which facilitates LPS-neutralization. In one embodiment, SEQ ID No. 8 comprises an LPS-/glycosaminoglycan binding motif, e.g., within region A having SEQ ID No. 3. In one embodiment, SEQ ID No. 9 comprises an LPS-/glycosaminoglycan binding motif, e.g., within region A having SEQ ID No. 3 and/or within region I having SEQ ID No. 5. In one embodiment, SEQ ID No. 10 comprises an LPS-/glycosaminoglycan binding motif, e.g., within region A having SEQ ID No. 3 and/or within region I having SEQ ID No. 5. In one embodiment, SEQ ID No. 11 comprises an LPS-/glycosaminoglycan binding motif, e.g., within region A having SEQ ID No. 3 and/or partially within region I having SEQ ID No. 5. In one embodiment, SEQ ID No. 12 comprises an LPS-/glycosaminoglycan binding motif, e.g., within region A having SEQ ID No. 3 and within region II having SEQ ID No. 6. In one embodiment, SEQ ID No. 13 comprises an LPS-/glycosaminoglycan binding motif, e.g., partially within region A having SEQ ID No. 3 and/or partially within region II having SEQ ID No. 6. In one embodiment, the term "partially within" is meant to be understood as a part of a first sequence or an entire first sequence overlapping with a part of a second sequence and/or region or with an entire second sequence and/or region. In one embodiment, SEQ ID No. 14 comprises an LPS-/glycosaminoglycan binding motif, e.g., within region B having SEQ ID No. 4 and/or within region III having SEQ ID No. 7. In one embodiment, SEQ ID No. 15 comprises an LPS-/glycosaminoglycan binding motif, e.g., within region B having SEQ ID No. 4 and/or within region III having SEQ ID No. 7. In one embodiment, SEQ ID No. 16 comprises an LPS-/glycosaminoglycan binding motif. In one embodiment, SEQ ID No. 17 comprises an LPS-/glycosaminoglycan binding motif. In one embodiment, SEQ ID No. 18 comprises an LPS-/glycosaminoglycan binding motif.

In one embodiment, a sequence of SEQ ID No. 1 comprises a sequence having any of SEQ ID No. 2-18. In one embodiment, a sequence of SEQ ID No. 2 comprises a sequence having any of SEQ ID No. 3-15.

In one embodiment, a sequence of SEQ ID No. 20 is, for example, occurring in region II of hoBPI and acBPI. In one embodiment, a sequence of SEQ ID No. 21 is, for example, a sequence of any of SEQ ID No. 8-14. In one embodiment, a sequence of SEQ ID No. 22 is, for example, a sequence of SEQ ID No. 15. In one embodiment, a sequence of SEQ ID No. 23 is, for example, a sequence of any of SEQ ID No. 16-17. Without wishing to be bound by any theory, the inventors believe that any of SEQ ID No. 2-23, preferably SEQ ID No. 3-23, more preferably SEQ ID No. 3-7 and/or 8-15, even more preferably SEQ ID No. 3-15, are highly relevant for the LPS-neutralizing efficiency of BPI. In one embodiment, a protein, e.g., BPI or a fusion protein, comprising, preferably exposing, a sequence of any of SEQ ID No. 2-23, preferably SEQ ID No. 3-23, more preferably SEQ ID No. 3-7 and/or 8-15, has therapeutic activity against a sepsis associated with an infection with Gram-negative bacteria. In one embodiment, the presence, preferably exposure, of an amino acid sequence having any of SEQ ID No. 3-7 and/or 8-15, facilitates and/or enhances a therapeutic activity against a sepsis associated with an infection with Gram-negative bacteria, e.g., by facilitating and/or enhancing neutralization of LPS. In one embodiment, BPI for use of the invention alleviates a sepsis associated with an infection with Gram-negative bacteria by neutralizing LPS.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±50%, ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g., "a", "an" or "the", this includes a plural of that noun unless something else is specifically stated.

### BRIEF DESCRIPTION OF THE FIGURES

The present invention is now further described by reference to the following figures.

All methods mentioned in the figure descriptions below were carried out as described in detail in the examples.

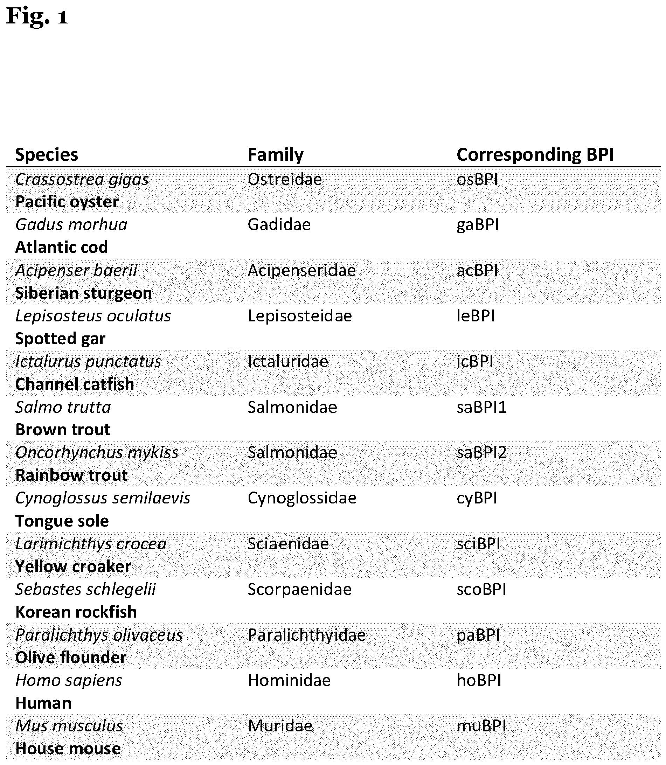

**Figure 1** shows a list of different examples of BPI orthologues. Named BPIs are representatives of their families. The listed non-human BPIs, BPIs of other members of the named families, and fragments thereof, can be used in a method of preventing or treating a sepsis associated with an infection with Gram-negative bacteria. The inventors have demonstrated that scoBPI is particularly useful in preventing or treating a sepsis.

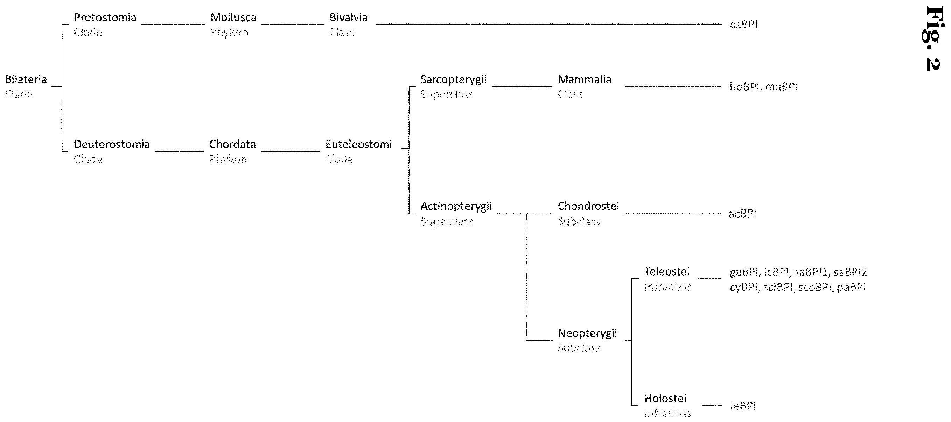

**Figure 2** shows a cladogramm of different examples of BPI orthologues. Herein osBPI represents Protostomia, Mollusca and Bivalvia BPI; muBPI represents Sarcopterygii and Mammalia BPI; acBPI represents Chondrostei BPI; gaBPI, icBPI, saBPI1, saBPI2, cyBPI, sciBPI, scoBPI and paBPI represent Teleostei; leBPI represents Holostei BPI; gaBPI, icBPI, saBPI1, saBPI2, cyBPI, sciBPI, scoBPI, paBPI, leBPI represent Neopterygii BPI; acBPI, gaBPI, icBPI, saBPI1, saBPI2, cyBPI, sciBPI, scoBPI, paBPI, leBPI represent Actinopterygii BPI; muBPI, acBPI, gaBPI, icBPI, saBPI1, saBPI2, cyBPI, sciBPI, scoBPI, paBPI, leBPI represent Deuterostomia, Chordata and Euteleostomi BPI. The listed BPIs are representatives of their families, infraclasses, subclasses, classes, superclasses, phyla and clades. The named non-human BPIs or fragments thereof as well as other non-human BPI members of the named families, infraclasses, subclasses, classes, superclasses, phylums and clades or fragments thereof can be used in a method of preventing or treating a sepsis associated with an infection with Gram-negative bacteria.

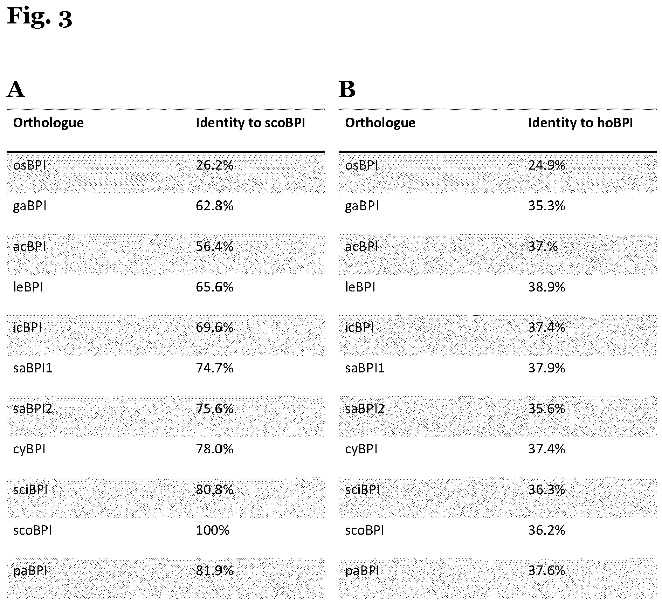

**Figure 3** shows the sequence identity of different examples of BPI orthologues as compared to scoBPI and hoBPI. (A) Sequence identity of different examples of BPI orthologues selected from other Actinopterygii BPIs to scoBPI is in the range of from 50% to 85%. Sequence identity of different examples of BPI orthologues selected from other Teleostei BPIs to scoBPI is in the range of from 60% to 85%. (B) Sequence identity of different examples of BPI orthologues selected from Actinopterygii BPIs to hoBPI is below 40%. Sequence identity of scoBPI to hoBPI is about 36.5%.

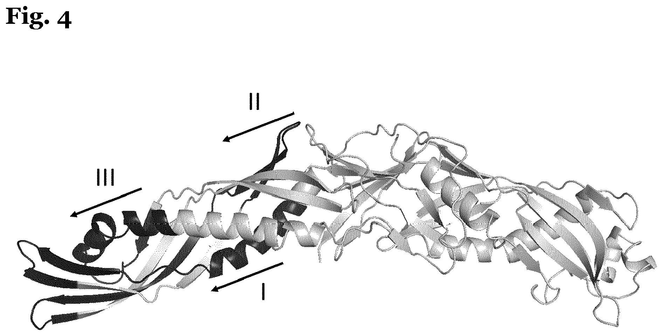

**Figure 4** shows functional regions I, II and III. Functional regions of human BPI were selected in analogy to [3] and are depicted in black. In scoBPI region I contains aminoacids 17 to 50, region II aminoacids 65 to 100 and region III aminoacids 142 to 168. All three regions contain sequences that neutralize lipopolysaccharide [3]. Region II also harbors a peptide sequence with bactericidal activity.

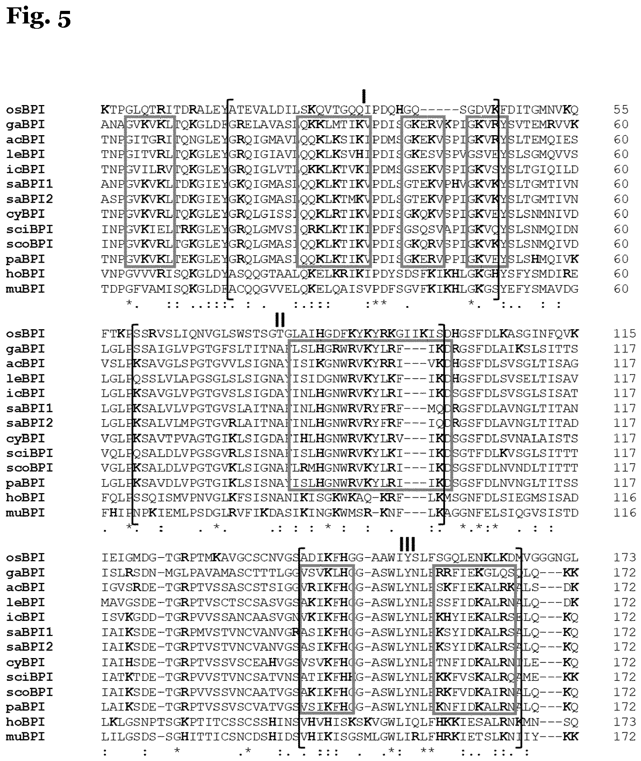

**Figure 5** shows a comparison of the number of positively charged aminoacids (K, R, H) in region I to III of different BPI orthologues. Regions are marked by brackets. Clusters of positively charged aminoacids in Actinopterygii BPIs are marked with grey boxes. Basic aminoacids are highlighted in bold.

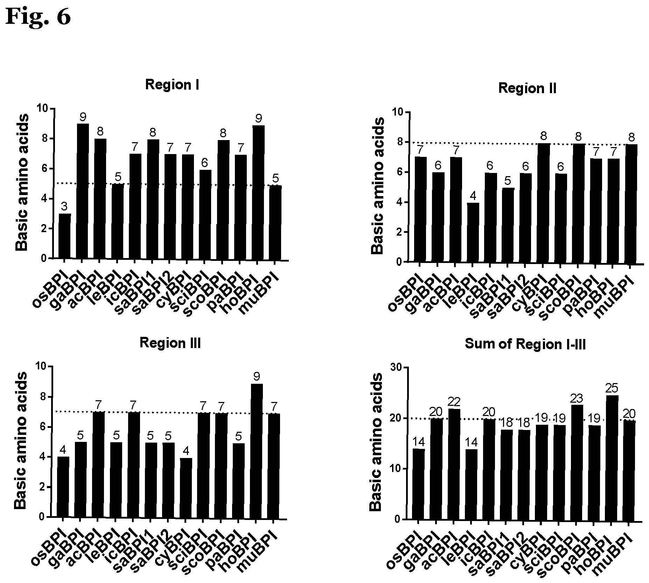

**Figure 6** shows numbers of positively charged aminoacids (K, R, H) in region I to III of different BPI orthologues. Graphs depict numbers of positively charged aminoacids in region I to III. As depicted, acBPI and scoBPI show surprisingly high numbers of positively charged aminoacids within said regions. All examples of Actinopterygii BPIs contain the same or even a higher number of positively charged aminoacids than muBPI.

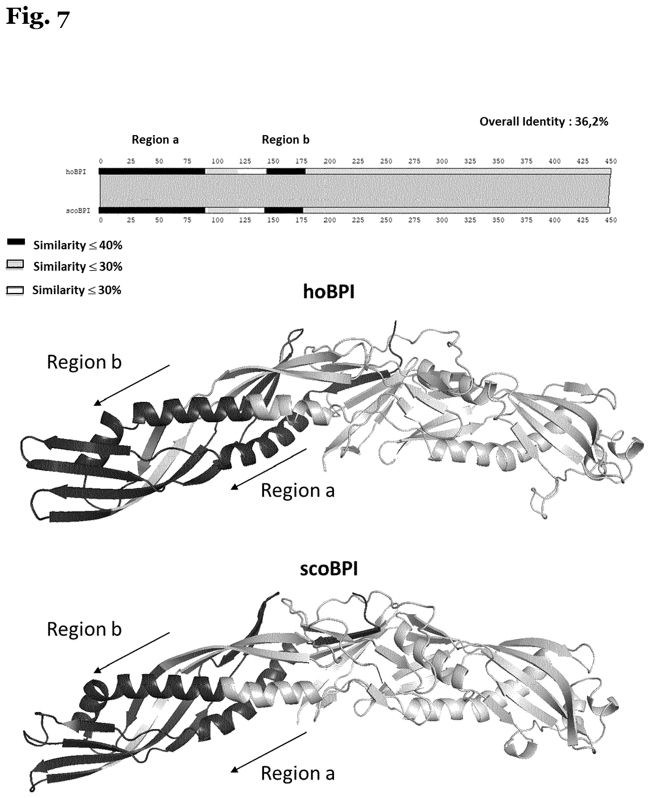

**Figure 7** shows functional regions a and b. Functional regions of human BPI were determined by sequence comparison of hoBPI and scoBPI using SIM-alignment tool showing an overall identity of 36.2%. The identity is higher in region a and b whereas the identity is lower in the remaining regions of the protein when compared to overall identity. In scoBPI region a contains aminoacids 1 to 102 and region b aminoacids 140 to 178. Region a and b contain sequences that neutralize lipopolysaccharide. Region a also habors a peptide sequence with bactericidal activity.

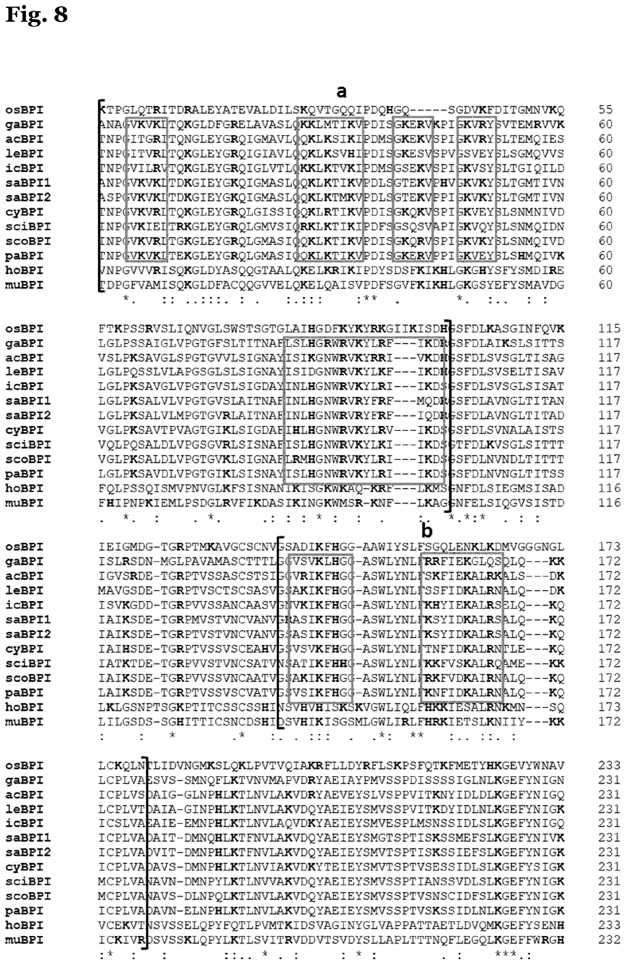

**Figure 8** shows a comparison of the number of positively charged aminoacids (K, R, H) in region a and b of different BPI orthologues. Regions are marked by brackets. Clusters of positively charged aminoacids in Actinopterygii BPIs are marked with grey boxes. Basic aminoacids are highlighted in bold.
   osBPI: *Crassostrea gigas*
   cyBPI: *Cyonoglossus semilavis*
   gaBPI: *Gadus morhua*
   acBPI: *Acipenser baerii*
   leBPI: *Lepisosteus oculatus*
   icBPI: *Ictalururs punctatus*
   saBPI1: *Salmo trutta*
   saBPI2: *Oncorynchus mykiss*
   sciBPI: *Larimichtys crocea*
   paBPI: *Paralichthys olivaceus*
   scoBPI: *Sebastes schlegelii*
   hoBPI: *Homo sapiens*
   muBPI: *Mus musculus.*

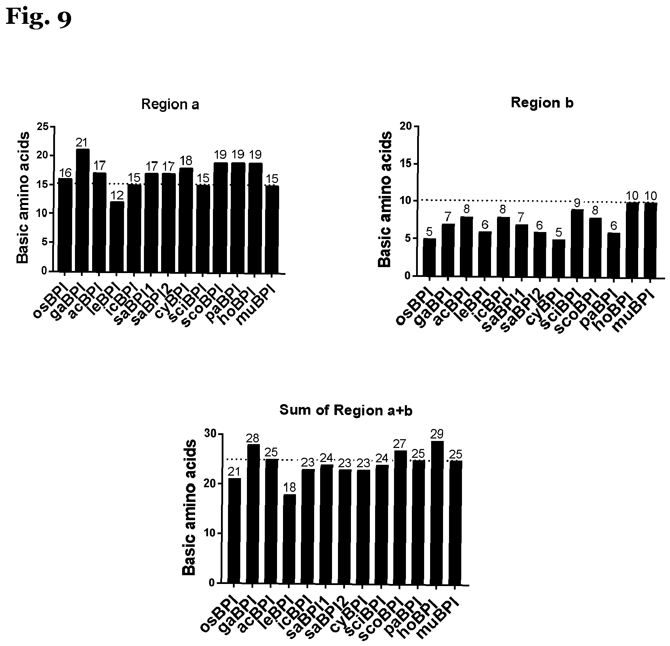

**Figure 9** shows the numbers of positively charged aminoacids (K, R, H) in region a and b of different BPI orthologues. The graphs depict numbers of positively charged aminoacids in region a and b. As depicted, gaBPI and scoBPI show surprisingly high numbers of positively charged aminoacids within said regions. All examples of Actnopterygii BPIs, except for leBPI, contain the same or even a higher number of positively charged aminoacids than muBPI in region a.

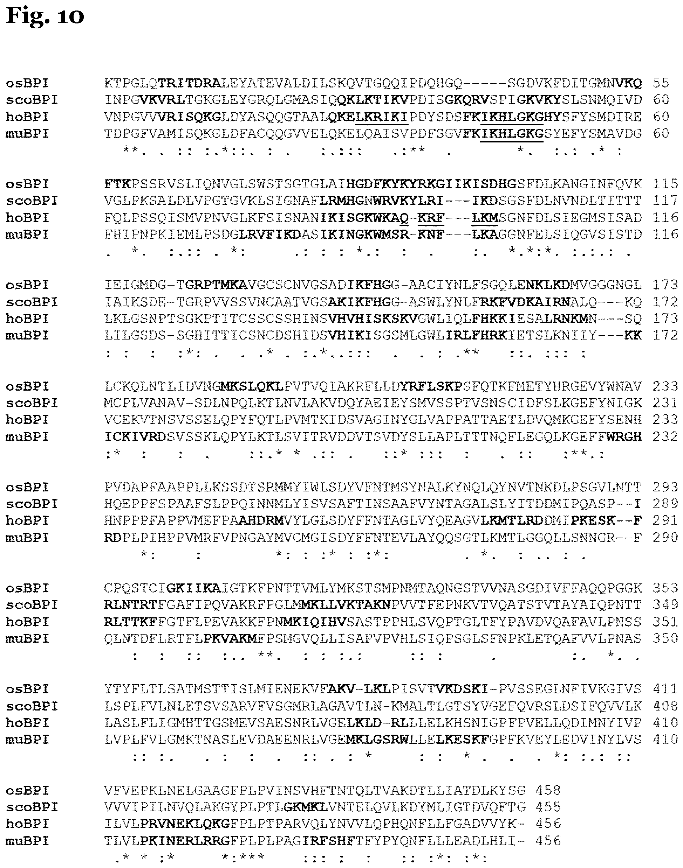

**Figure 10** shows LPS- and glycosaminglycan-binding motifs in comparison of osBPI, scoBPI, hoBPI and muBPI. Glycosaminglycan-binding motifs are marked in bold. LPS-binding motifs are marked in bold and are underlined. Both LPS- and glycosaminglycan-binding motifs are capable to bind and neutralize LPS. In comparison to muBPI and osBPI, scoBPI and hoBPI show clusters of LPS- and glycosaminglycan-binding motifs within the cationic tip of BPI. Without wishing to be bound by any theory, the inventors believe that clusters of LPS- and glycosaminglycan-binding motifs within the cationic tip of BPI play a role in the increased LPS-neutralizing efficiency of scoBPI and hoBPI compared to muBPI and osBPI.

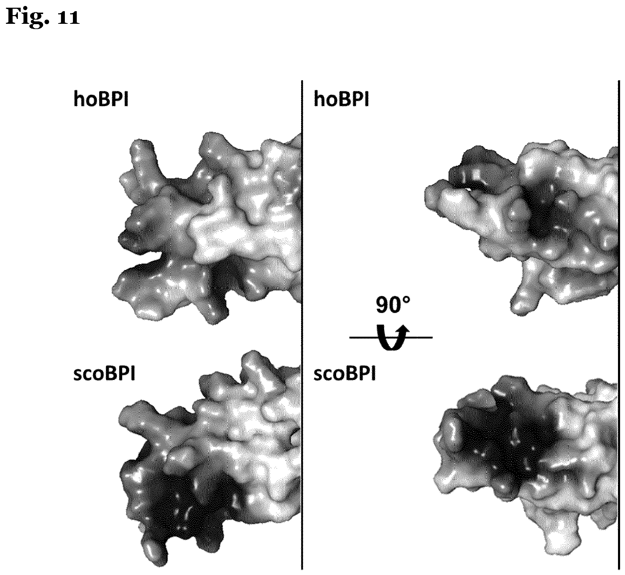

**Figure 11** shows positive charged aminoacids at the cationic tip of BPI. The cationic tip is defined by three loops. Loop 1 contains aminoacids 21 to 55, loop 2 aminoacids 81 to 107 and loop 3 aminoacids 138 to 177 as exemplified for scoBPI. The three loops contain sequences that neutralize lipopolysaccharide. The positive charge of the cationic tip of hoBPI and scoBPI is depicted and, surprisingly, the positive charge at the tip of BPI in scoBPI is more prominent than in hoBPI. Without wishing to be bound by any theory, the inventors believe that the positive charge at the tip of scoBPI plays a role in the increased LPS-neutralizing efficiency of scoBPI compared to hoBPI.

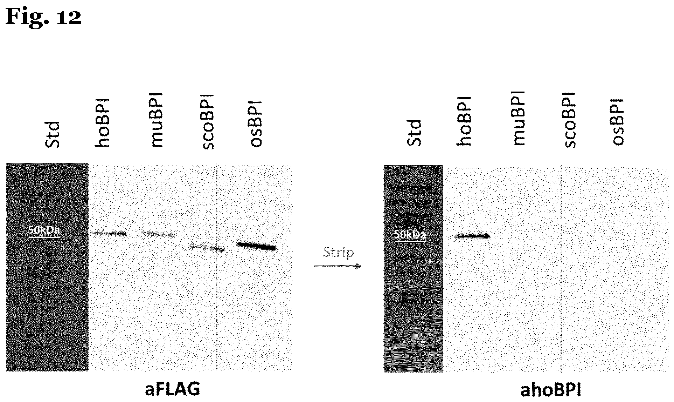

**Figure 12** shows a detection of expressed orthologues in western blot analysis. Orthologues are detected by an anti-flag-antibody (left) C-terminally integrated in the proteins (left). Only hoBPI is detected by the polyclonal anti-BPI-antibody directed against hoBPI (right) since no cross reactivity is present of the polyclonal anti-BPI-antibody with muBPI, scoBPI or osBPI.

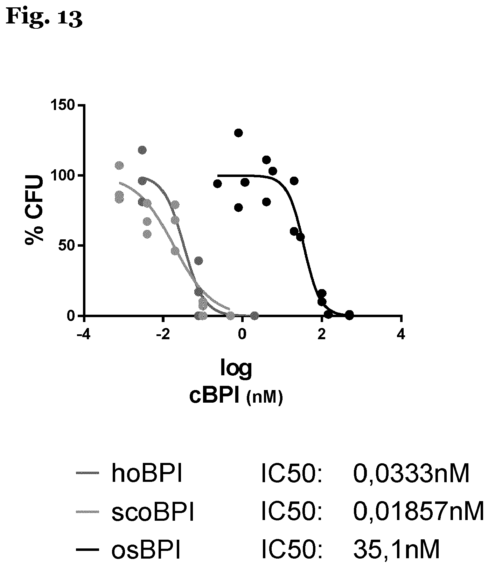

**Figure 13** shows a comparison of the bactericidal activity between hoBPI, scoBPI and osBPI. All tested orthologues kill *Escherichia coli* DH10B. A higher amount of osBPI (in nM) is needed in comparison to hoBPI and scoBPI as indicated by IC50 (concentration of 50% growth inhibition). It is shown that scoBPI has a higher bactericidal activity towards *Escherichia coli* DH10B than hoBPI.

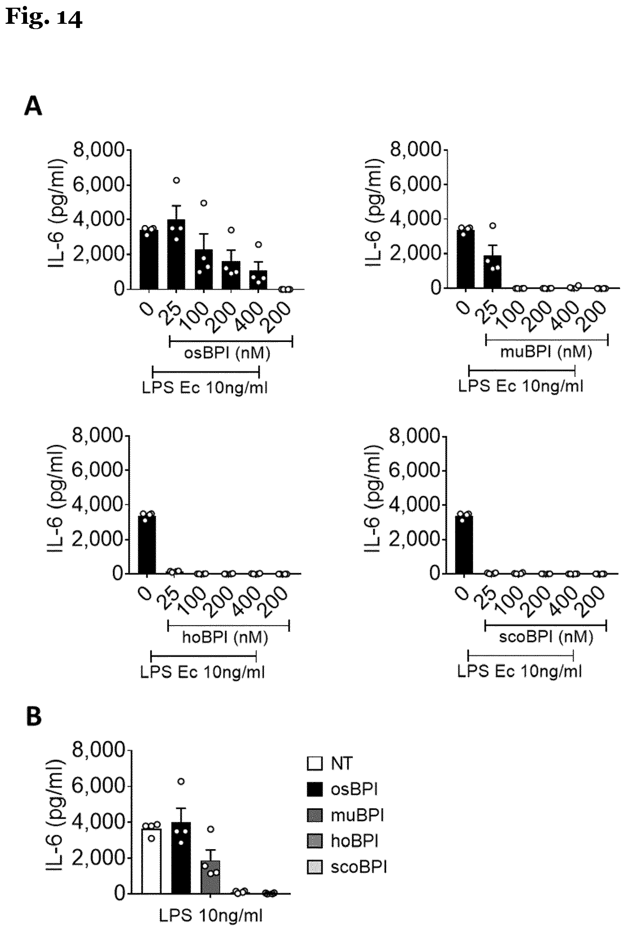

**Figure 14** shows a comparison of the LPS-neutralizing capacity between hoBPI, muBPI, scoBPI and osBPI. All tested orthologues neutralize the pro-inflammatory properties of LPS after stimulation of peripheral blood mononuclear cells. The LPS-neutralizing capacity of osBPI is lower in comparison to muBPI. The efficiency of osBPI and muBPI is lower in comparison to hoBPI and scoBPI. (A) LPS-neutralizing capacity of osBPI, muBPI, hoBPI and scoBPI is depicted as exemplified for IL-6 at 24 h after stimulation with 10 ng/ml LPS. The inventors demonstrated that scoBPI has the highest efficiency of the tested BPIs. (B) LPS-neutralizing capacity of osBPI, muBPI, hoBPI and scoBPI and are compared at a concentration of 25 nM BPI as exemplified for IL-6 at 24 h after stimulation with 10 ng/ml LPS.

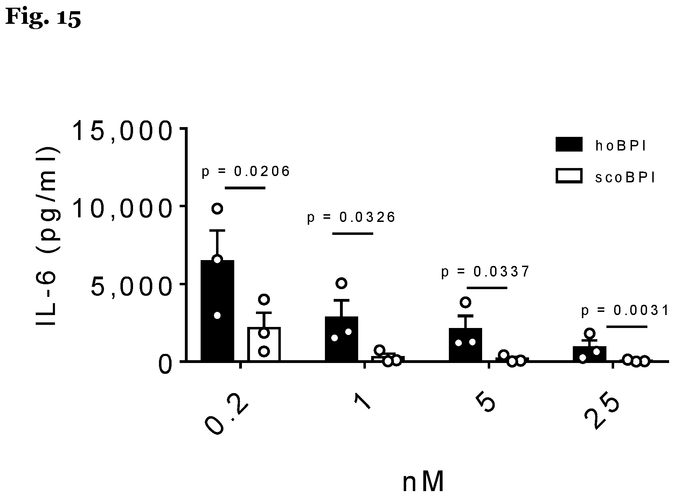

**Figure 15** shows a comparison of the LPS-neutralizing capacity between hoBPI and scoBPI. PBMCs were stimulated with 10 ng/ml LPS for 40 h resulting in a medium IL-6 level of 20,000 pg/ml. Both scoBPI and hoBPI neutralized the potency of LPS as exemplified for IL-6. However, scoBPI was more potent in neutralization of IL-6 as compared to hoBPI.

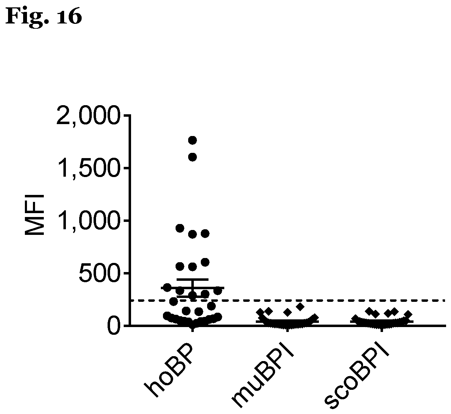

**Figure 16** shows that serum of SLE patients contains antibodies which bind to hoBPI but not scoBPI or muBPI. Serum of SLE patients was incubated with beads coated with BPI orthologues. Binding of anti-BPI antibodies within the serum was detected by an anti-Fc antibody. Around 43% of sera derived from SLE patients contain aBPI-antibodies which are known to neutralize BPI functions such as LPS-neutralizing or bactericidal capacity. The detected anti-BPI-antibodies of SLE patients did not react with muBPI and scoBPI. The presence of anti-BPI-antibodies in SLE sera indicate antibody-mediated decrease of BPI functions including LPS-neutralizing or bactericidal capacity. The absence of reactivity of these anti-BPI-antibodies in SLE sera with BPI orthologues, as exemplified for muBPI and scoBPI, indicates that the functions BPI orthologues are not affected by anti-BPI-antibodies derived from SLE patients or other patients with diseases associated with anti-BPI-antibodies. Thus, non-human BPI is particularly useful in preventing or treating sepsis in patients that have anti-BPI antibodies, such as SLE patients.

In the following, reference is made to the examples, which are given to illustrate, not to limit the present invention.

### EXAMPLES

### Example 1: Methods

### Sequence alignment

The protein sequences were selected from UniProt. Sequence alignments were blasted using the Clustal Omega program of the European Bioinformatics Institute.

### Expression and purification of recombinant BPI orthologues

Templates for recombinant BPI were designed by flanking the BPI sequence without its native signal-peptide with an N-terminal HA sequence and a C-terminal FLAG tag. Human and murine BPI were obtained by standard cloning techniques into pCR3 Vector. Ostreid BPI was synthesized by GeneArt and inserted into a pcDNA3.1 (+) expression vector. HEK293T cells were transfected using the ExpiFectamine293TM Transfection Kit (#A14525, Gibco). Recombinant hoBPI and scoBPI was purified by ion chromatography on a HiTrap SP HP column (#17-1151-01, GE Healthcare) followed by size-exclusion chromatography. Recombinant Murine BPI was purified by ion chromatography followed by affinity chromatography on an anti-Flag M2 (Sigma Aldrich) coupled NHS-activated HP column (GE Healthcare). osBPI was purified by affinity chromatography on an anti-Flag column followed by size exclusion chromatography.

### Western Blotting

The purified recombinant BPI orthologues were diluted in PBS to a concentration of 250nM and loaded onto a FastCast 12% stain-free gel (#1610185, Bio-Rad). Electrophoresis was conducted at 220V for 30 minutes. Proteins were then blotted onto a nitrocellulose membrane using the Trans-Blot Turbo TRA Transfer Kit (#170-4270, Bio-Rad). After protein transfer, membranes were blocked in 5% non-fat dried milk TBS-T for 1 hour. Incubation of primary antibodies was done overnight at 4°C followed by three washes with TBS-T for 10 minutes. Secondary antibodies were incubated for 45 minutes at room temperature with three consequent washing steps. Membranes were then incubated with a working solution of Clarity Western ECL substrate (#1705061, Bio-Rad) for five minutes. Chemiluminescence was detected with the digital imaging system Chemi Lux Imager (Intas).

### Killing assay

*E. coli* DH10B were cultured in LB medium at 37°C until the oD600 equaled 0,4. The *E. coli* cells were further diluted in PBS with 0,01% Tween20 (PBS-T) and incubated with recombinant BPI or PBS-T as mock control for one hour at 37°C. Dilutions of *E. coli* were then plated onto blood agar plates and incubated at 37°C overnight. Colony forming units (CFU) were quantified manually the next day.

### Stimulation of human PBMCs

For isolation of human PBMCs, blood was collected from healthy volunteers after informed consent by using heparinized tubes (Li-Heparin-Gel-Monovette, Sarstedt, Nümbrecht, Germany). The blood was centrifuged in leucosep tubes containing Ficoll-Paque^{™} PLUS (Cytiva Europe GmbH, Freiburg, Germany) at 1,000 × g for 10 min. The interphase containing the leukocytes was collected and subsequently washed twice with RPMI 1640. The PBMC pellet was then resuspended in AIM V^{™} (# 12055091, ThermoFisher Scientific) and cell number was determined. Next, 1×10^5 PBMCs were seeded into 96 well TC plate (#83.3924, Sarstedt) and rested for 3 hours before stimulation. Stimulants were diluted in AIM V^{™} and pre-incubated in protein LoBind^{®} tubes (#0030108116, Eppendorf) for 30 minutes at 37°C prior to stimulation of PBMCs. PBMC supernatants were collected after 24 hours and stored at -20°C for cytokine analysis.

### Quantification of human cytokines

Levels of human interleukin (IL)-6 and tumor necrosis factor (TNF) were quantified by Luminex^{®} technology (Austin, TX, USA). Capture and detection antibodies for IL-6 were from the OptEIA^{™} Set for human IL-6 (#555220, BD) and capture and detection antibodies for human TNF were from (#555212, BD). Signals were detected by Streptavidin R-Phycoerythrin LumiGrade Ultrasensitive Reagent (#05351693103, Roche) and cytokine concentrations were quantified by Human Cytokine 25-Plex Protein Standard (#LHC0009M, Invitrogen).

### Example 2: vertebrate versus invertebrate BPI

Gene sequences encoding BPI are well conserved throughout the eukaryotic domain and their derived proteins are highly similar in structure. Therefore, the inventors considered various orthologues to human BPI, including both a closely and distantly related orthologue to human BPI, for a new therapeutic approach against Gram-negative sepsis. Expression of two bactericidal orthologous proteins was described for the pacific oyster *Crassostrea gigas,* an aquatic invertebrate. The inventors have found that C. *gigas* ostreid BPI (osBPI) has poor bactericidal effects against *E. coli* and have demonstrated that human BPI (hoBPI), teleostBPI (scoBPI) and murine BPI (muBPI), but not osBPI prevent activation of human PBMCs by E.coli-derived LPS. The results suggest that teleost BPIs are better-suited candidates for adjuvant therapy of Gram-negative infection than human BPI. Thereby, scoBPI is of special value in patients showing a functional deficit in BPI or in patients with a BPI deficiency.

### Example 3:

Sequence comparison of Actinopterygii BPI revealed an increased number of positively charged aminoacids in the cationic tip as well as the defined regions I and region a of BPI as exemplified for acBPI, gaBPI, icBPI, saBPI1, saBPI2, cyBPI, sciBPI, scoBPI, paBPI and leBPI. Highest numbers of basic aminoacids were especially seen within examples of Holostei BPI such as acBPI and Teleosti BPI such as gaBPI, icBPI, saBPI1, saBPI2, cyBPI, sciBPI, scoBPI and paBPI.

### Example 4:

Without wishing to be bound by any theory, the inventors believe that the surface charge of a BPI orthologue determines its LPS-neutralizing capacity, i.e., surface charge is of high relevance and occurs at clusters of LPS- and glycosaminglycan-binding motifs and regions with high numbers of positively charged aminoacids. Thereby, the surface charge is higher when the positive charge of positively charged aminoacids is directed towards the surface and not towards the core of BPI. Depiction of the surface charge of scoBPI in comparison to hoBPI shows that scoBPI displays a higher positive charge on its surface, particularly at the cationic tip of scoBPI as opposed to hoBPI. Given the sequence homology of scoBPI with other Actinopterygii BPIs and especially with other Teleostei BPIs, other Actinopterygii BPIs and especially other Teleostei BPIs show LPS-neutralizing capacity comparable to scoBPI.

### Example 5:

Surface exposure of positive charges at the cationic tip of BPI is highly beneficial for preventing or treating Gram-negative sepsis. The inventors showed that picomolar concentrations of scoBPI are capable of neutralizing the proinflammatory capacity of LPS after stimulation of PBMCs. Surprisingly, scoBPI was superior to hoBPI in its neutralization capacity even at concentrations as low as 200 pM. Therefore, scoBPI is superior to hoBPI for the therapy of Gram-negative sepsis.

### Example 6:

Anti-BPI-antibodies in sera of SLE patients react with hoBPI. However, no reactivity of anti-BPI-antibodies in sera of SLE patients with scoBPI was observed. Further evidence is provided since the polyclonal anti-BPI-antibody directed against BPI did not bind scoBPI as shown by western blot analysis. Without wishing to be bound by any theory, the inventors believe that the low level of identity in protein sequences and corresponding epitopes are responsible for lack of detection of scoBPI by antibodies directed against hoBPI. Therefore, scoBPI can be therapeutically used in patients with anti-BPI-antibodies which have been shown to impair the function of BPI such as its LPS-neutralizing capacity or its bactericidal function.

### REFERENCES

[1] Dentener, M. A. et al. Antagonistic effects of lipopolysaccharide binding protein and bactericidal/permeability-increasing protein on lipopolysaccharide-induced cytokine release by mononuclear phagocytes. Competition for binding to lipopolysaccharide. Journal of immunology (Baltimore, Md. : 1950) 151, 4258-4265 (1993).
[2] Levin, M. et al. Recombinant bactericidal/permeability-increasing protein (rBPI21) as adjunctive treatment for children with severe meningococcal sepsis: a randomised trial. The Lancet 356, 961-967; 10.1016/S0140-6736(00)02712-4 (2000).
[3] Little, R. G. et al. Functional Domains of Recombinant Bactericidal/Permeability-Increasing Protein (rBPI23). The Journal of Biological Chemistry. 1994, Vol. 269, No. 3, pp. 1865-1872.
[4] Beamer, L. J. et al.. Crystal structure of human BPI and two bound phospholipids at 2.4 angstrom resolution. Science. 1997 Jun 20;276(5320):1861-4. doi: 10.1126/science.276.5320.1861. PMID: 9188532.

## Claims

1. Bactericidal/Permeability Increasing Protein (BPI) for use in a method of preventing or treating a sepsis associated with an infection with Gram-negative bacteria, wherein said BPI is non-human BPI or a fragment thereof.

2. BPI for use according to claim 1, wherein said BPI is non-human vertebrate BPI or a fragment thereof; preferably is teleost BPI or a fragment thereof.

3. BPI for use according to claim 1 or 2, wherein said BPI is BPI of a *Sebastinae* sp. or a fragment thereof; preferably BPI of a *Sebastes* sp. or a fragment thereof; more preferably is BPI of *Sebastes schlegelii* or a fragment thereof.

4. BPI for use according to any one of the foregoing claims, wherein said BPI comprises or consists of an amino acid sequence of SEQ ID No. 1.

5. BPI for use according to any one of the foregoing claims, wherein said BPI or fragment thereof comprises an N-terminal fragment of BPI, preferably an N-terminal fragment of BPI having an amino acid sequence of SEQ ID No. 2.

6. BPI for use according to any one of the foregoing claims, wherein said BPI or fragment thereof comprises an amino acid sequence of any of SEQ ID No. 3-18.

7. BPI for use according to any one of the foregoing claims, wherein said BPI comprises a modification selected from
- an amino acid substitution at position 347 of SEQ ID No. 1, preferably an asparagine at position 347 being substituted with alanine;
- one or more amino acid substitutions providing a sequence of any of SEQ ID No. 19-23;
- a modified glycosylation pattern, preferably a removed glycosylation e.g. at position 347 of SEQ ID No. 1; wherein, optionally, said glycosylation pattern is modified by a mild acid treatment, an amino acid substitution, and/or an enzymatic treatment, preferably a glucosidase treatment e.g. using mannosidase;
and a combination thereof.

8. BPI for use according to any one of the foregoing claims, wherein said sepsis is associated with
- a presence of Gram-negative bacteria or component(s) thereof, preferably lipopolysaccharide, in a bloodstream of a patient;
- a dysfunction, preferably failure, of one or more organs of said patient; and/or
- a septic shock.

9. BPI for use according to any one of the foregoing claims, wherein, in said method, said BPI is administered to a patient in need thereof; preferably administered systemically or locally; more preferably administered intravenously, intravascularly, orally, nasally, mucosally, intrabronchially, intrapulmonarily, intradermally, subcutaneously, intramuscularly, intravascularly, intraperitoneally, intrathecally, intracerebral, intracranial, intraocularly, intraarticularly, intranodally, intratumorally, and/or intrametastatically.

10. BPI for use according to claim 9, wherein said patient is **characterized by** BPI deficiency, e.g. a decreased BPI level and/or a functional BPI deficit; neutropenia; defective neutrophil function; and/or by increased levels of anti-neutrophil cytoplasmic antibodies, preferably anti-BPI autoantibodies.

11. BPI for use according to claim 9 or 10, wherein said patient is a newborn, a toddler, or a patient having an age of at least 40 years, preferably of at least 60 years.

12. BPI for use according to any one of claims 9-11, wherein said patient suffers from or is at risk of acquiring, in addition to said infection with Gram-negative bacteria, a disease selected from cancer; neutropenia; infections other than said infection with Gram-negative bacteria, e.g. HIV infections; autoimmune diseases, e.g. systemic lupus erythematodes, psoriasis, rheumatic diseases such as rheumatoid arthritis, granulomatosis with polyangiitis, and inflammatory bowel diseases such as Crohn's disease and ulcerative colitis; hematologic diseases; cystic fibrosis; COPD; alpha-1 antitrypsin deficiency; bronchiectasis; TAP deficiency; primary biliary cirrhosis; vasculitis; and immune suppression, e.g. iatrogenic immune suppression and therapeutic immune suppression.

13. BPI for use according to any one of the foregoing claims, wherein, in said method, said BPI is co-administered with any of
- an antimicrobial agent, preferably selected from antibacterial agents, antifungal agents, virostatic agents, and antiparasitic agents, more preferably an antibiotic;
- an anti-inflammatory agent, preferably selected from steroids, non-steroidal antiphlogisitic agents, and antibodies targeting a pro-inflammatory cytokine or an immune receptor;
- an immunosuppressive agent, preferably selected from cylosporin, tacrolimus, mycophenolat, and methotrexate;
- an immunotherapeutic agent, preferably selected from anti-TNF antibodies, immune checkpoint inhibitors, TLR ligands, and C-type lectin ligands, e.g. glucans;
and combinations thereof.

14. BPI for use according to any one of the foregoing claims, wherein said BPI neutralizes lipopolysaccharide.

15. BPI for use according to any one of the foregoing claims, wherein said BPI alleviates said infection and/or said sepsis by neutralizing a lipopolysaccharide of said Gram-negative bacteria.
